# EUROPEAN PATENT APPLICATION

(11) **EP 4 763 267 A1**
(43) Date of publication of application: **24.06.2026**
(21) Application number: 24221209.0
(22) Date of filing: 18.12.2024
(51) Int. Cl.: A61M 60/161, A61M 60/289, A61M 60/427, A61M 60/468, A61M 60/191, A61M 60/47, A61M 60/515, A61M 60/569, A61M 60/839, A61M 60/865

(54) **CARDIAC ASSIST DEVICES AND SYSTEM, AND METHODS FOR IMPLEMENTING THE SAME**

(71) Applicant: Zotz, Rainer, 54634 Metterich (DE)
(72) Inventor: Zotz, Rainer, 54634 Metterich (DE)
(74) Representative: KIPA AB

(57) **Abstract**

A cardiac assist device and system are disclosed and an exemplary method of using the same, the cardiac assist device comprising a steering line, and a catheter for guiding said steering line. The catheter being configured to be advanced percutaneously through vasculature to be positioned along a longitudinal axis of a heart outside a pericardium of said heart inside a patient's body. The steering line being adapted to be received through the catheter and being advanced therethrough, substantially along the longitudinal axis of the heart. The steering line being configured to form a closed loop extending from the catheter, thereby forming a cardiac loop for positioning around the heart, so that it substantially straddles the heart, wherein the cardiac loop is configured to move between a first position and a second position, wherein in said first position the cardiac loop has a substantially enlarged profile, thereby enabling the heart to receive blood, and wherein in said second position the cardiac loop has a substantially reduced profile, enabling the heart to contract, thereby assisting the heart to pump blood, thereby mechanically assisting cardiac function, thereby simulating the natural movement or contractile rhythm of the heart.

## Description

### TECHNICAL FIELD

The present disclosure relates to a mechanical means for treating heart failure. More specifically, it relates to a mechanical cardiac assist device for use in assisting and supplanting the mechanical function of the heart.

### BACKGROUND

Heart failure affects approximately 12.5 million people in Germany alone, and many more worldwide. In Germany it is the most common reason for hospitalization and death. In heart failure the heart muscle is unable to pump blood as well as it should. The heart can become enlarged, and it can leave the heart too weak and/or stiff to fill and pump blood properly. As a result, blood can back up and fluid can build up in the lungs, causing shortness of breath. Conventional treatments rely on drug therapies to improve survival only to a limited extent and can only rarely be used to treat acute events. Defibrillation devices are common. However, these devices only treat heart malfunction due to rhythmic events, related to dysfunction in the electrical rhythm of the heart but fail to provide intervention where heart function is affected due to a mechanical failure in the heart, such as in heart failure. Today different cardiac augmenting methods and devices are known, such as cardiopulmonary resuscitation (CPR) and a ventricular assist device (VAD). When CPR is performed, it can lead to complications that may need to be rectified. Common complications due to CPR are rib fractures, sternal fractures, bleeding in the anterior mediastinum, heart contusion, hemopericardium, upper airway complications, damage to the abdominal viscus, fat emboli, pulmonary complications - pneumothorax, hemothorax, lung contusions and so on. Additionally, in order to augment the heart with CPR over a longer time it preferably requires a number of people to conduct the CPR with a similar force due to each person becoming physically tired. Further, the CPR needs to be performed when a person is laying down on his/her back on a solid ground. However, CPR can only be used in acute events, even though the mechanism of action of CPR may be desirable for use in patient's in heart failure, for assisting heart function over both a shorter and/or longer period of time. Thus, there is a need in the art for a device that provides similar functionality as CPR but can be used over a variable period of time as needed in heart failure, without the complications listed herein above.

VADs are sometimes intended for short term use, typically for patients recovering from heart attacks or heart surgery, while others are intended for long-term use, typically for patients suffering from advanced congestive heart failure. VADs are designed to, mechanically by pumping, assist either the right (RVAD) or left (LVAD) ventricle, or both ventricles at once (BiVAD). Known VADs are however constructed as complex devices which are quite intricate to implant into a patient. Thus, VADs are time consuming to implant and require surgeons and other highly trained clinical personnel to implant them. Further, since the VADs are connected directly to the blood stream for pumping the blood they also have common associated problems for devices with blood contact such as coagulation associated problems. Also, in some VADs the blood may be passed externally from the body to a pump positioned outside the body and then returned to the body, which may increase the risk of infection. The VADs also have the problem of having moving parts, in particular supported by bearings, i.e. parts which can fail and need to be replaced, which is complicated in an implanted device like a VAD. Thus, there is a need in the art for a mechanical assist device that prevents the risks associated with VAD and/or pumps, including risk of infection, and risk associated with having one or more parts of the VAD being located outside the body. A disadvantage of VAD's are that they require opening of the chest. Additionally, it is difficult to synchronize the function and of the RVAD and LVAD, and there is yet no perfect synchronization of RVADs and LVADS in the same patient.

US 6,254,525 to Reinhardt et al., discloses a cardiac assist system that includes a sensor that is operative to sense the electrical activity of a patient's heart. The sensed electrical activity is processed to determine the onset of ventricular contraction. A compressive force from a ventricular assist device is applied to the ventricles during at least a portion of the heart's ventricular contraction. The cardiac assist system also includes an electrical stimulating system. Thus, in the event the heart begins to fibrillate, the electrical stimulating system is actuated to apply stimulating pulses to the heart. The present device is a complicated device that requires input from sensors about the heart activity and responding thereafter using the ventricular assist device that is implanted within the patient. The electrode may be mounted on or placed against an inside surface of the ventricular assist device for reliable placement against the patient's heart. Thus, the ventricular assist device is a bulky device positioned inside the patient. It can be difficult to fit and/or position inside the cardiac cavity and may additionally abrade or damage the pericardium with its position and/or during use. Thus, a simplified cardiac assist device is needed that is easy to deploy and position inside the cardiac cavity, percutaneously adjacent the heart and is safe to use against the pericardium.

US 8,509,894 to Forsell, discloses an implantable device for improving the pump function of the heart of a human patient by applying an external force on the heart muscle is provided. The device comprises at least one heart contacting organ. The heart contacting organ is adapted to be movable to change the position of said force exerted on the heart after the implantable device has been implanted in the human patient. The disclosure further relates to a method of using said device. However, the device is a mechanical device that has moving parts and rotatable parts close to and configured to engage and contact the heart. This can complicate the device, increasing risk of failure because of the many moving parts. It may also cause risk of damage to the pericardium and/or inadvertent bruising of the heart muscle because of the movement of the multiple parts. Additionally, the device is large and/or cumbersome to implant and requires surgical implantation, which involves surgically creating a large opening in the chest. Thus, there is a need for a mechanical cardiac assist device in the art, that can be deployed without open surgical means and that minimizes the impact from contact.

US 2008/0064917 to Bar et al., A1 discloses an apparatus that is provided for compressing at least one ventricle of a heart. The apparatus includes a plurality of inflatable elements and a pump in fluid communication with the inflatable elements. At least one band is in mechanical communication with the inflatable elements. A portion of the band is adapted to be placed around at least a portion of the heart in mechanical communication with the portion of the heart. The inflatable elements are arranged such that when the inflatable elements are inflated by the pump, the inflatable elements apply more force to the heart via shortening of the portion of the band than via expansion of the inflatable elements against the heart. Since the band itself is typically of substantially fixed length, the remainder of the band that is not surrounding the given shape-changing member is shortened. This shortening of the remaining portion of the band applies a compressive force to the heart, supporting the function of the heart by ejecting blood therefrom during systole. However, the device is complicated to implement as it requires synchronous use of multiple shape-changing members and a band. Thus, even if one of the shape-changing members fails, it can alter the amount of compressive force on the heart. Thus, there is a need in the art of a simpler device that is easy to deploy and can compress the heart as desired using a simple mechanical device.

US 6,808,483 to Oritz, discloses heart support and assist devices for supporting and assisting the pumping action of the heart. Various examples include mesh support devices, devices using straps, spiral shaped devices, catheter-based devices and related methods. Oritz discloses an inflatable bladder where Inflation and subsequent deflation of bladder will contract left ventricle against the support provided by internal support member comprising a rigid or simi-rigid plate or metallic material to expel blood and subsequent deflation will allow left ventricle to expand and refill with blood. Thus, Oritz discloses an array of complicated structures that surround the heart, where the bladder disclosed must work in conjunction with a rigid support member to contract the left ventricle. Thus, there is a need in the art to provide a simpler mechanical device that is easier to implement.

WO 2016/079159 A1 to Zotz, discloses a radially compressible cardiac gripper for at least mechanical stimulation of the heart. The cardiac gripper has two gripper arms, wherein at least one of the gripper arms comprises a flexible section configured for movement of the arm having the flexible section. However, it is difficult to deploy the cardiac gripper percutaneously, and thus may require open surgery to implant the device. Thus, there is a need in the art for a mechanical cardiac assist device that can be deployed into the heart minimally invasively without the use of surgical intervention.

Thus, the problem as contemplated by the inventors of the present disclosure, is that despite available drug therapies and lifestyle interventions, the treatment of heart failure remains challenging. Many patients do not respond adequately to treatment and there is a need for innovative solutions that can effectively support cardiac function. Heart failure affects millions of people worldwide. In the United States alone, approximately 6.2 million adults live with this condition. In Europe there are around 15 million people. The prevalence increases with age, leading to an increase in cases given the aging population in many countries. Heart failure is a chronic condition in which the heart is unable to pump enough blood to meet the body's needs. This leads to an insufficient supply of oxygen and nutrients to the organs and tissues, which can have serious health consequences. Heart failure can be caused by various factors such as coronary artery disease, high blood pressure, heart valve defects or previous heart attacks. Patients with heart failure often experience symptoms such as shortness of breath, fatigue, swelling in the legs, and fluid retention in the body. These symptoms significantly affect their quality of life and limit their ability to carry out everyday activities. Heart failure is associated with high morbidity and mortality. The 5-year survival rate after diagnosis is only about 50%, making it one of the deadliest chronic diseases. The treatment and management of heart failure places a significant economic burden on healthcare systems and patients. Approximately $30.7 billion is spent annually in the United States on the care of patients with heart failure. This includes hospital stays, medications and doctor visits. In Europe the costs amount to around 2% of total healthcare expenditure. Despite available drug therapies and lifestyle interventions, the treatment of heart failure remains challenging.

### SUMMARY

The inventors of the present disclosure have developed a cardiac assist device and system that provides a solution for the above noted problem. The inventors have developed an innovative cardiac support system specifically designed to support the function of weak right and left hearts and significantly improve the quality of life of patients with heart failure. It is a mechanical support system designed to support the pumping function of the heart and improve blood flow in the body.

The examples of the present disclosure of the device and system as proposed by the inventors provide advantages as outlined herein. Some of the features and benefits of the cardiac assist device and system of the present disclosure include one or more of: (i) Advanced cardiac function support in the form of mechanical support, wherein the device and/or system of the present disclosure, partially or completely takes over the pumping function of the heart, which reduces the strain on the weakened heart and ensures adequate blood flow to the organs; (ii) Biocompatibility, wherein the system uses biocompatible materials to ensure optimal compatibility; adaptable design with modular components, wherein the system's modular components allow for easy adjustment to patient size and maintenance; (iii) Easy implementation and handling using minimally invasive implantation, wherein the device and/or the system can be implanted minimally invasively, shortening recovery time and reducing the risk of complications, (iv) User-friendly controls, wherein the system provides an intuitive user interface and user-friendly control unit enable easy handling and monitoring by medical personnel; Reliability and longevity, wherein the device and/or the system have a substantially robust design, wherein the device and/or system is designed to last and/or provides reliable support over a substantially long period of time; (iv) Advanced Monitoring and Adjustment, wherein the system and/or device provides an automatic adjustment, wherein in some examples the device and/or the system automatically adjusts to the patient's ECG to ensure optimal support; (v) Improved quality of life for increased mobility, wherein the system's portable design and compact size may allow patients to continue their daily activities with minimal restrictions; (vi) Reduced symptoms, wherein by effectively supporting heart function, symptoms of heart failure such as shortness of breath and fatigue may be substantially reduced.

Thus, the inventors of the present disclosure have developed a medical device that supports and/or assists and/or replaces the contraction of the heart and/or heart muscle. The device of the present disclosure may additionally provide one or more of the following additional advantages: it does not comprise moving parts and/or mechanical moving parts; provides minimal contact with the surface of the heart and thus pericardium; can be deployed minimally invasively without open surgery (i.e. using percutaneous access through a blood vessel to be deployed outside the pericardium inside the chest cavity; and provides contact with the heart and thus the pericardium using a substantially blunt atraumatic and/or rounded surface of the device.

The device as contemplated by the inventors adapts to the respective needs of the defective heart (and/or heart muscle). In some examples, the device as envisioned by the present inventors, does not provide permanent and/or direct contact with the blood circulation when the device is used for long-term use. Thus, there is no direct connection and/or communication between the device and the outside environment, which can pose a major risk of infection.

As envisioned by the inventors, the device can be used and/or implanted in a patient in a (small) hospital setting with a catheter laboratory. The procedure to deploy the device is percutaneous and does not require cardiac surgery or surgical intervention. The device may support both the left and the right heart and may result in fewer complications. In some examples the device comprises an external and/or an internal motor and is a mechanical resuscitation device.

In accordance with a method of the present disclosure, a device is disclosed that mechanically compresses the heart by mimicking the natural movement of the heart during systole and diastole.

In accordance with natural movement of the heart, the muscle fibers of the heart surround hollow cavities that are filled with blood. As a result, their contraction produces a pressure within the cavity and this pressure drives the flow of blood. The period of contraction of the heart muscle, specifically the ventricles, is called systole which creates pressure in the ventricles and sends blood to the lung from the right ventricle and to the body from the left ventricle. The period of relaxation of the ventricles is called diastole, where oxygenated blood enters the left ventricle from the left atria and deoxygenated blood enters the right ventricle from the right atria.

The device and/or system of the present disclosure mimic the contraction of the cardiac muscles that is normally caused by the electrical pathways of the heart, namely, one or more of: the sinoatrial node, atrioventricular node, Bundle of His and the Purkinje fibers. The Purkinje fibers are located in the inner ventricular walls of the heart and allows the heart's conduction system to allow the ventricles to contract and maintain a consistent heart rhythm.

In some examples, the device of the present disclosure comprises a steering line loop or in other words a cardiac loop, that positioned around the heart, preferably around the ventricles, that contracts mimicking movement of the muscle fibers of the heart to mimic contraction, thus creating pressure within the ventricles, in order to drive the flow of blood as above in systole. Thereafter the loop relaxes, thereby allowing the heart muscles to relax, thus mimicking diastole, allowing blood to fill the ventricles as above. In some examples, the device or instrument (the contractor, or the cardiac loop), of the present disclosure can be advanced through veins as well as arteries. The entrance into the body can also be made from the vein or subclavian artery (left shoulder) or the cervical veins/cervical arteries. In some examples, the action of the cardiac loop, mainly concerns the transverse shortening of the heart (i.e. in the short axis). Both hearts are supported, i.e. both the left heart and the right heart function are supported by the cardiac loop. In other examples, the cardiac loop functions to contract the heart by providing both a transverse force (i.e. along the short axis) as well as a longitudinally compressive force (i.e. along the long axis of the heart) away from the apex of the heart.

The cardiac muscle is responsible for the contractility of the heart, and therefore the pumping action. The cardiac muscle must contract with enough force and enough blood to supply the metabolic demands of the entire body.
Thus, the device of the present disclosure allows for mimicking the natural movement of the cardiac muscle and thus the pumping action of the heart, through mechanical means.

It is an object of the present disclosure to provide an improved system for mechanical stimulation of the heart.

According to a first aspect of the present disclosure a cardiac assist device is disclosed for use in assisting mechanical stimulation of a heart. The cardiac assist device comprises a steering line adapted to be pushed out via the catheter such that the steering line forms a substantially closed loop extending from an opening at the distal end of the catheter, said closed loop defining a cardiac loop, wherein at least one end of the steering line is arranged through an opening at the proximal end of the catheter for controlling the movement of the cardiac loop at the distal end.

The cardiac assist device provides for a minimally invasive approach that allows for heart access without the need for opening the chest or using a heart-lung machine, which minimizes patient trauma and recovery time.

The cardiac assist device allows for a procedure to arrange the cardiac loop around a heart and then pulling at least one end of the line loop (or in other words a steering line loop or cardiac loop) for pumping augmentation of the heart. By not making any intrusion into the heart itself, the risk of contamination is minimized as well.

According to an additional or alternative aspect of the present disclosure, both ends of the steering line are arranged to be controlled at the proximal end of the catheter.

A further option is also to push the at least one end of the line loop, or in other words the cardiac loop, after each pulling of the end of the at least one end of the line loop or cardiac loop. Generally, the heart will flex sufficiently to cope without the need for a push of the at least one end of the line loop or cardiac loop. However, by having this pushing, the risk of chafing is minimized.

Depending on the situation, the pulling of the line loop (or cardiac loop) end or ends could be performed manually and externally of the body.

Additionally or alternatively, the pulling of the at least one end of the line loop (or cardiac loop) is performed by a motor located inside or outside of the body.

According to a further aspect of the present disclosure, the steering line is hollow. Further, the steering line could have an inflatable part along the length of the steering line arranged to be forming the loop, thereby defining an inflatable cardiac loop. This may further minimize the risk of chafing between the line and/or cardiac loop and the heart.

According to an additional or alternative aspect of the disclosure as regards to minimizing the chafing, a flexible film is attached and rolled onto the steering line along at least a part of the steering line arranged to be forming the loop or in other words the cardiac loop.

In a further additional or alternative aspect of the present disclosure, the device and/or system of the present disclosure comprises a mechanical pumping mechanism, that is attached to the proximal end of the catheter and the steering line and arranged such that the steering line is at least pulled in an operating mode by the mechanical pumping mechanism. For instance, a spring loaded device could be used such that when manually compressed, the steering line is pulled, thereby compressing or squeezing the cardiac loop and when released it is biased with a spring to return to its initial position, thereby allowing the cardiac loop to move back to its original uncompressed position.

According to a preferred aspect of the device and/or system of the present disclosure, the pumping mechanism is a motor. A motor is preferred over the manual pumping should the pumping be required over an extended period of time. In order to be able to use the system in any place, the motor is according to an aspect of the present disclosure battery powered. Hence, in some examples, the system could be used even if there is a power shortage in the regular power supply.
According to a further aspect of the present disclosure the battery is adapted to be wirelessly charged. This is especially beneficial if the system, which according to a yet another aspect of the present disclosure, having a pumping mechanism adapted to be arranged inside a body.

The pumping mechanism is according to an additional or alternative aspect of the present disclosure adapted to be arranged outside of a body.
For the system to be used for various situations and bodies of different sizes and ages, the system, according to a further aspect of the present disclosure, comprises a control unit connected to the motor controlling the speed of the motor for controlling the pumping frequency.

On a similar note, the system according to a further additional or alternative aspect of the present disclosure further comprises a control unit connected to the motor controlling the amplitude of the pumping. Hearts vary in size and it is therefore beneficial to be able to control the amplitude of the pumping.

As an example of use, a method of implanting a system for mechanically augmenting a heart, comprises entering a catheter from a radial or cubital vessel, advancing the catheter through an opening punctured in a radial access opening or in a cubital vein towards the desired site, advancing a steering line arranged in a substantially closed loop, thereby forming a cardiac loop, from an opening at the distal end of the catheter, and arranging the cardiac loop around the apex of the heart. Optionally, the catheter could be further advanced through at least a part of a thoracic artery or the internal mammary artery.

Also optionally, the catheter and thus the device of the present disclosure is advanced through a punctured site in the left thoracic artery or the right thoracic artery or the internal mammary artery.

In accordance with the system mentioned above having a hollow steering line having at least a part that is elastic, the method of implanting the system further comprises inflating the elastic part of the loop.

For facilitating the understanding of the system, a method of augmenting a heart is described below.

The method of augmenting a heart comprises arranging a line loop or cardiac loop around a heart and pulling at least one end of the line loop or the cardiac loop for pumping augmentation of the heart.

Optionally, the method further comprises pushing the at least one end of the line loop or cardiac loop after each pulling of the end of the at least one end of the line loop or cardiac loop.

The pulling of the at least one end of the line loop or cardiac loop is performed manually and externally of the body according to an aspect of the present disclosure.

According to an additional or alternative aspect of the present disclosure the pulling of the at least one end of the line loop or cardiac loop is performed by a motor located inside or outside of the body. When the motor is placed internally, the motor may be placed similarly to a conventional pacemaker implanted in the subclavian area.

The system further comprises at least a part of the loop or in other words the cardiac loop comprises at least one sensor. For instance, it could be a heart sensor that senses the electrical activity of the heart and generates corresponding electrical signals. A controller could then be electrically connected to the sensor and to a control unit responsive to receipt of the electrical signals from the sensor to determine the frequency and/or amplitude of pumping.

Further features of, and advantages with, the present invention will become apparent when studying the appended claims and the following description. The skilled person realizes that different features of the present invention may be combined to create examples other than those described in the following, without departing from the scope of the present invention as defined by the claims.

### BRIEF DESCRIPTION OF THE DRAWINGS

The above, as well as additional objects, features and advantages of the present invention, will be better understood through the following illustrative and non-limiting detailed description of exemplary examples of the present disclosure, wherein:
Fig. 1A is a schematic view of a cardiac assist device and system according to an example of the present disclosure;
Fig. 1B is a schematic view of a method of implanting a cardiac assist device, according to an example of the present disclosure;
Fig. 1C is a schematic view of a cardiac assist device and system according to an example of the present disclosure;
Fig. 1D is a schematic view of a heart showing forces exerted on the heart, during natural contraction of the heart;
Fig. 1E is a schematic view of a cardiac assist device and system according to an example of the present disclosure showing forces exerted on the heart;
Fig. 1F and Fig. 1G show a location on the heart, of a preferred placement of the cardiac loop of a cardiac assist device, in accordance with an example of the present disclosure;
Fig. 2A, Fig. 2B, Fig. 2C, Fig. 2D and Fig. 2E, schematically show a cardiac assist device and/or system comprising a cardiac loop, and a method of performing a procedure using said cardiac assist device and/or system in accordance with an example of the present disclosure;
Fig. 3A shows an additional or alternative example of a cardiac loop comprising an inflatable cardiac loop in accordance with examples of the present disclosure;
Fig. 3B shows a further additional or alternative example of a cardiac loop comprising a film loop in accordance with additional examples of the present disclosure;
Figs. 4A, Fig. 4B, Fig. 4C, Fig. 4D and Fig. 4E, schematically show a cardiac assist device and/or system comprising an alternate example of a cardiac loop, and a method of performing a procedure using said cardiac assist device and/or system, in accordance with an example of the present disclosure;
Fig. 5A and Fig. 5B show two additional or alternative examples of a cardiac assist device in accordance with examples of the present disclosure, respectively;
Fig. 6A and Fig. 6B show two additional or alternative examples of a cardiac assist device accordance with examples of the present disclosure, respectively;
Figs. 7A, Fig. 7B, Fig. 7C, Fig. 7D, Fig. 7E, and Fig. 7F, schematically show the various stages of a method of implanting a cardiac assist device and/or cardiac assist system of Fig. 3B, according to examples of the present disclosure;
Fig. 8A and Fig. 8B, schematically show cardiac assist methods, in accordance with examples of the present disclosure; and
Fig. 9A and Fig. 9B, show alternate examples of a cardiac loop in accordance with examples of the present disclosure, having a substantially boat-shaped configuration.

### DETAILED DESCRIPTION OF EXAMPLES OF THE DISCLOSURE

The present invention will now be described more fully hereinafter with reference to the accompanying drawings, in which exemplary examples of the invention are shown. The invention may, however, be embodied in many different forms and should not be construed as limited to the examples set forth herein; rather, these examples are provided for thoroughness and completeness. Like reference characters refer to like elements throughout the description. The invention is set out in the appended claims.

### Device

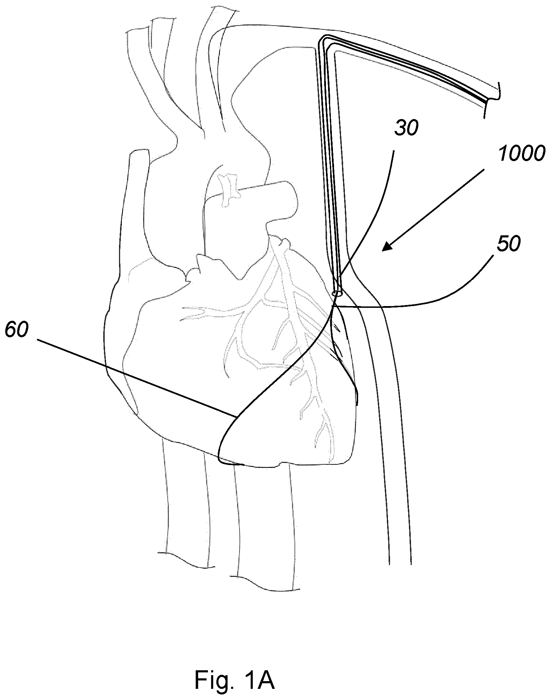

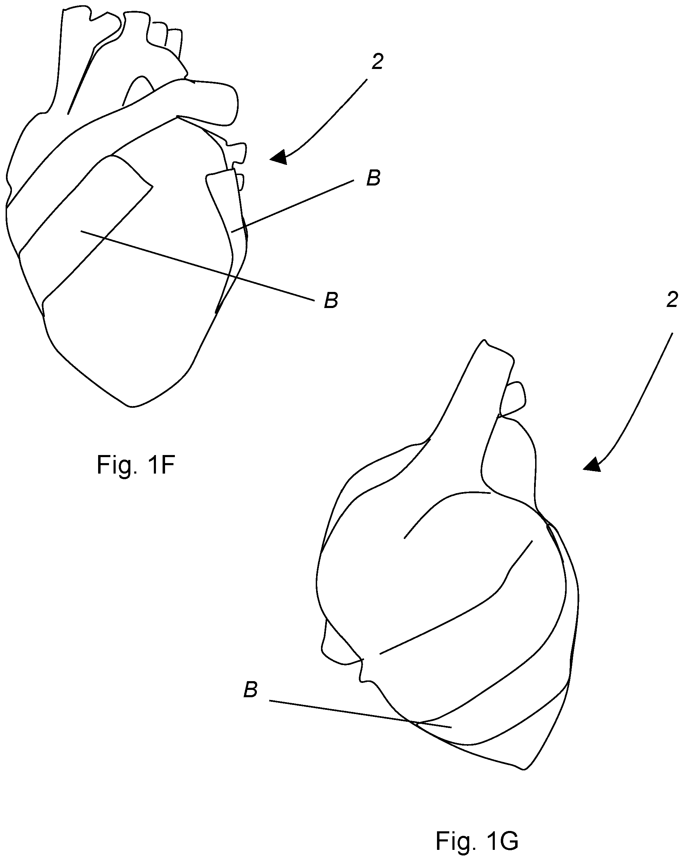

With reference to Figs. 1A-1C, a catheter device or a mechanical cardiac assist device 1000 is shown, for use in assisting mechanical stimulation of a heart 2 in a person 9000. The mechanical cardiac assist device 1000 comprises a steering line 50 and a catheter 30 having a distal end and a proximal end. The catheter 30, comprises an opening 3 at the distal end and an opening 32 at the proximal end. The steering line 50 is adapted to be inserted through the catheter 30 and configured to be advanced through the catheter 30 and/or pushed out via the distal opening 34 of the catheter 30. The steering line 50 is arranged such that the steering line 50 forms a substantially closed loop extending from the distal opening 34 at the distal end of the catheter 30. The substantially closed loop in accordance with examples of the present disclosure defines a cardiac loop 60.

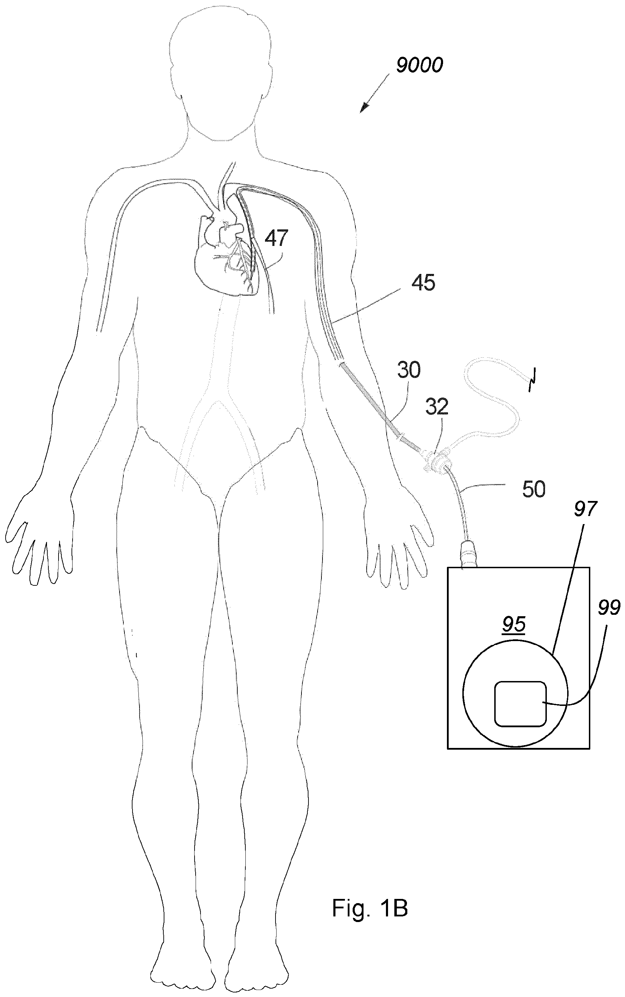

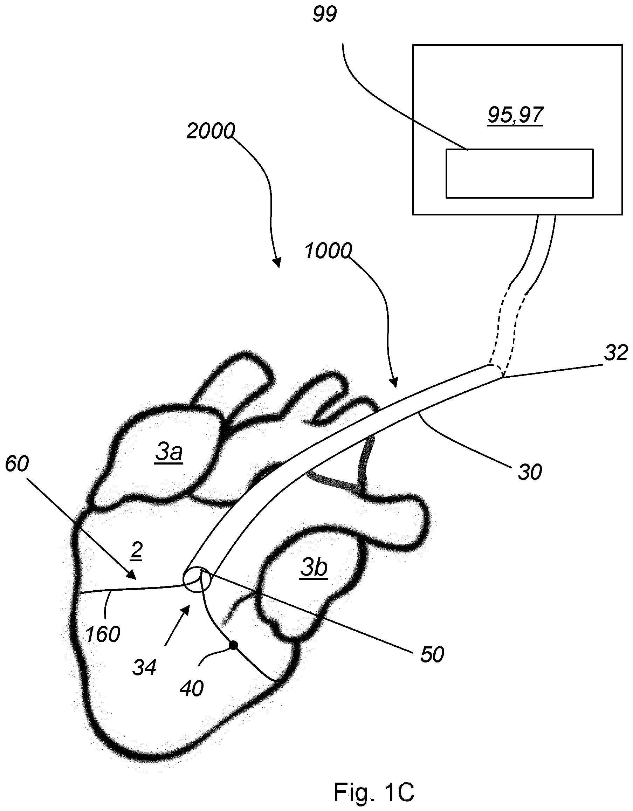

Thus, in some examples of the present disclosure a cardiac assist device 1000 is disclosed, with reference again to Fig. 1A and Fig. 1C. The cardiac assist device comprises a steering line 50 and a catheter 30 for guiding said steering line 50. The catheter 30 being configured to be advanced percutaneously through vasculature to be positioned along a longitudinal axis of a heart 2 outside a pericardium of said heart 2, inside a patient's body, shown in Fig. 1B. The steering 50 line being adapted to be received through the catheter and being advanced therethrough, substantially along said longitudinal axis of said heart 2. In some examples, as shown, a cardiac assist system 2000 is disclosed comprising a cardiac assist device 1000 and a controller 95 for controlling the same. In the example shown, the catheter 30 is advanced through the radial vein or artery 45 and advanced into one more branches of the thoracic and/or the mammary vein 47 and into the thoracic cavity to be placed adjacent the heart 2. In other examples, the catheter 30 may be advanced through a vein or an artery. Examples include the following but are not limited thereto: the subclavian vein or artery, jugular vein, carotid artery, femoral and brachial vein or artery, and advanced into one more branches, of the thoracic and/or the mammary vein 47 and into the thoracic cavity to be placed adjacent the heart 2.

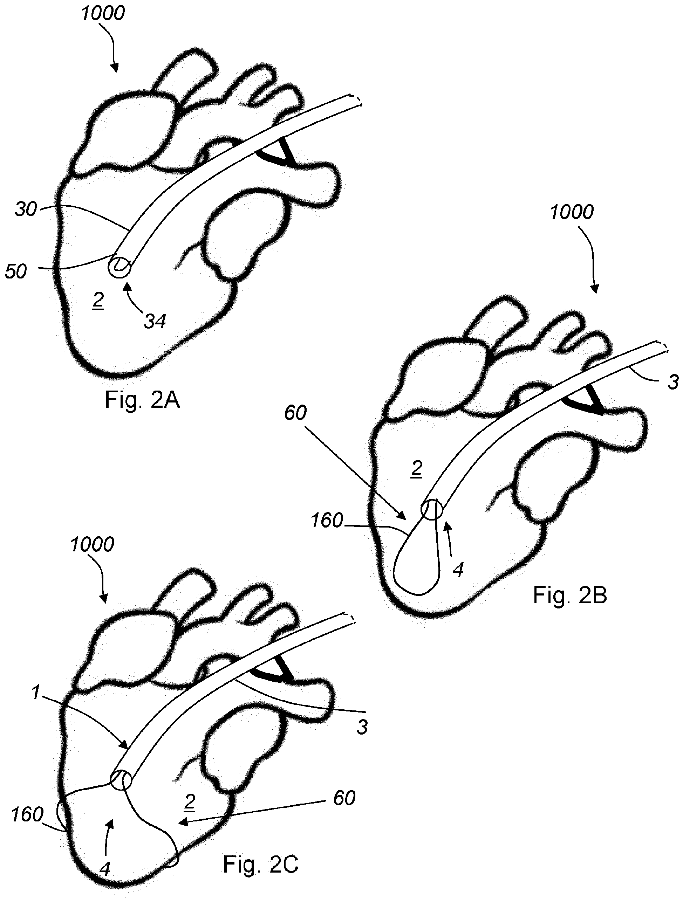

The steering line or guidewire 50 is configured to be advanced through the catheter 30 as shown, to be positioned adjacent to the heart 2. In some examples the steering line is configured to being arranged such that the steering line 50 forms a closed loop extending from the distal opening 34 of the catheter 30, thereby forming a cardiac loop 60 for positioning around the heart 2, so that it substantially straddles the heart 2. In some such examples, as shown in Fig. 1B, the controller 95 comprises a mechanical pumping mechanism or oscillator 97 for controlling the movement of the steering line 50 and/or the cardiac loop 60. In one such example, the mechanical pumping mechanism or oscillator 97, comprise a motor 99. In some such examples, said cardiac loop 60 is configured to move between a first position 62 and a second position 64 , wherein in said first position 62 the cardiac loop has a substantially enlarged profile, thereby enabling the heart 2 to receive blood (for examples into the ventricles 4a, 4b), and wherein in said second position 64 the cardiac loop 60 has a substantially reduced profile, enabling the heart 2 to contract, thereby assisting the heart 2 to pump blood, thereby mechanically assisting cardiac function and simulating the natural movement of the heart, as further illustrated in Figs. 2A-2E as described herein below according to the methods of the present disclosure. In some examples, the steering line 50 comprises a guidewire. In other examples, the steering line 50 in combination with the catheter or guiding catheter 30 forms or comprises a Bowden cable, as is known in the art. A Bowden cable is a type of flexible cable that is used to transmit mechanical force or energy by the movement of an inner cable (such as the steering line 50 in present disclosure) relative to a hollow outer cable housing (such as the catheter or guiding catheter 30 of the present disclosure). The advantages of Bowden cables or push-pull control cables are high efficiency, and reliable push/pull actuations that can be triggered even in the smallest dimensions. Due to the construction, a substantially strong leverage effect can be triggered by a low expenditure of force. Thus, in accordance with some examples of the present disclosure, said steering line 50 in combination with said catheter or guiding catheter 30, comprises a Bowden cable. In some such examples of the present disclosure, all of these materials and the materials for the examples disclosed herein have been carefully selected to be biocompatible for a substantially long or longer period of time.

In some such examples, the cardiac loop 60 mechanically assists the heart 2 by enabling the ventricles 4a, 4b to pump blood. In some examples said catheter 30 defines a proximal opening at a proximal end of the catheter 30 and a distal opening 34 at a proximal end of the catheter 30. The steering 50 line is adapted to be received through the proximal opening 32 and advanced through the distal opening 34 of the catheter 30, substantially along said longitudinal axis of said heart 2. In some examples of the present disclosure, the cardiac loop 60 comprises a steering line loop 160, as shown in Figs. 1A-1C. The steps of the method of the present disclosure using the cardiac assist device 1000 as described in Figs 1A-1C, are discussed further herein with respect to Figs. 2A-2E.

In some examples of the present disclosure, with reference to Figs. 1A-1F, 2A-2E and Figs. 4A to 4E, the cardiac assist device 1000 and or system 2000 of the present disclosure, through mechanical manipulation of the steering line 50 and/or the cardiac loop 160 mechanically mimics the contraction of the cardiac muscles that is normally caused by the electrical pathways of the heart, namely, one or more of: the sinoatrial node, atrioventricular node, Bundle of His and the Purkinje fibers. The Purkinje fibers are located in the inner ventricular walls of the heart and allows the heart's conduction system to create synchronized contraction of its ventricles and are essential for maintaining a consistent heart rhythm. The electrical signal in the heart travel from the Sinoatrial (SA) Node to the Atrioventricular (AV) node, through the Bundle of His (atrioventricular bundle) to the Purkinje fibers. Thus, examples of the present disclosure provide a cardiac assist device and/or system where the mechanical movement or manipulation of the steering loop or cardiac loop mimics the natural contraction of the heart muscle caused by electrical pathways in the heart, (namely one or more of the SA node, AV node, Bundle of His and the Purkinje fibers. In the case of a partially failing heart, in order to synchronize the cardiac assist device and/or system or the cardiac loop thereof to the natural contraction of the heart, an R-wave triggering of the ECG is used. This ECG is taken either from the skin with electrodes on the skin or from inside the body e.g. from sensors on the cardiac loop itself.

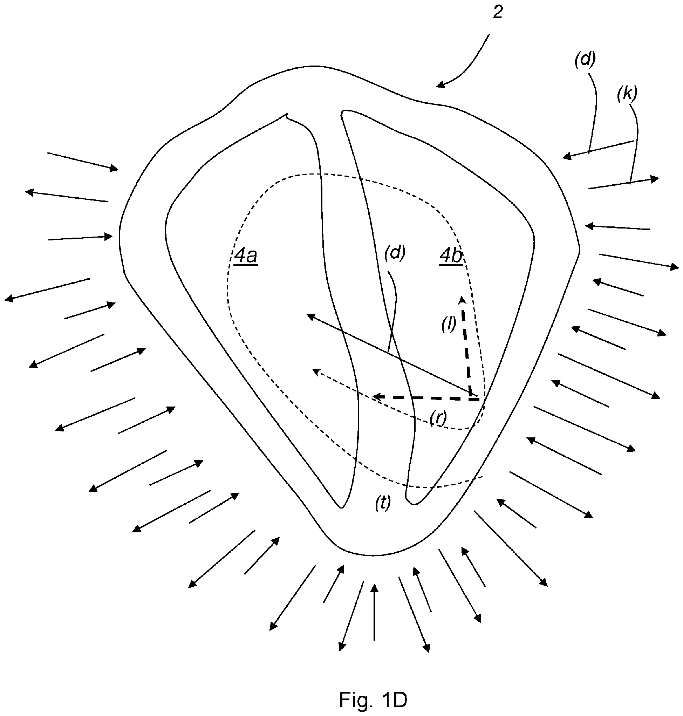

In a normal heart, heart muscle contraction occurs due to the sliding in of acting and myosin microfilaments so that the length of the sarcomere decreases. Sarcomere is the basis unit of heart muscle, and each muscle fiber has multiple sarcomeres. Contraction occurs when a stimulus transmitted through the Purkinje fibers depolarizes the muscle fiber. The force exerted by the heart during normal contraction of the ventricles 4a, 4b is such as shown in Fig. 1D. The force of contraction is represented by the force vector (d) formed by the ventricular muscles 2a, where the force vector (d) has both a longitudinal or axial force component (I) and a radial force component (r). An example of a force vector (d) is shown in Fig. 1D, showing the longitudinal/axial and radial force components (I) and (r). As shown in Fig. 1D, there is an opposite force exerted by the heart muscles, or specifically the ventricles 4a, 4b of heart 2, as represented by a reverse force vector (k), with force components being in the opposite direction to that of force vector (d), both longitudinally/axially, when the heart 2 relaxes into its normal relaxed state, when the ventricles 4a, 4b are filling with blood. Furthermore, another contractile force or in other words a third action of the heart after (or in addition or during the) short 14 and long axis 12 shortening is the squeezing action by the heart muscle, to pump blood which in one example may be similar to like squeezing a towel.

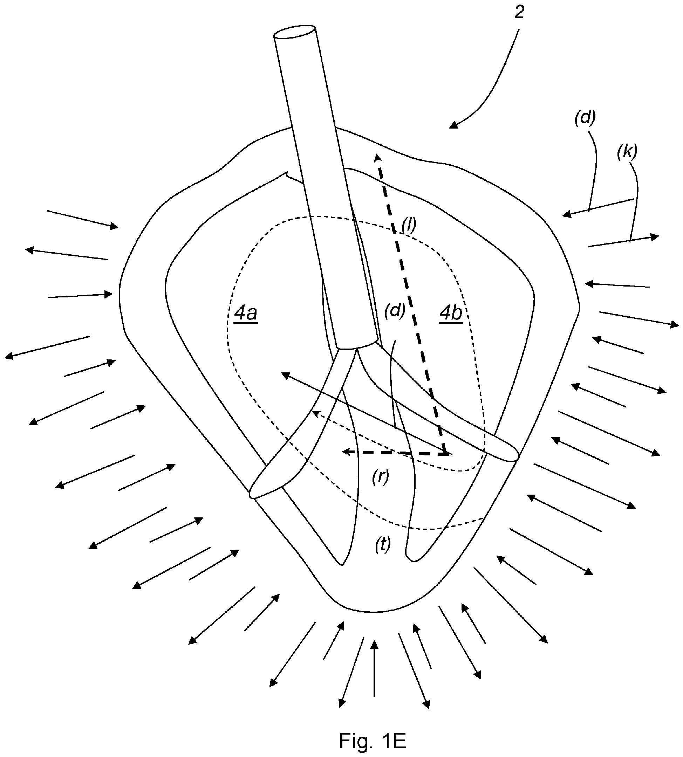

The cardiac assist device 1000 and/or system 2000 of the present disclosure mimics or simulates the force vector (d) created by the ventricles 4a, 4b of the heat 2 during normal contraction of the heart muscle 2a. The cardiac loop 60 of the device and/or system of the present disclosure itself contracts as it is pulled both along the longitudinal axis 12 of the heart 2, thereby creating a longitudinal force component (I), and is contracted radially inwards along the short axis 14 of the heart, thereby creating a radial force component (r), thereby mimicking the force vector (d) formed by the natural contraction 4a, 4b of the ventricles, as shown in Fig. 1E. The cardiac loop 60 of the present disclosure exerts a compressive contractile force on the heart muscle that is axially upwards along the long axis 12 of the heart, and radially inwards along the short axis 14 that mimics the natural ventricular contraction of the heart. This allows the heart 2 to contract and pump blood from the heart 2 to the body and to the lungs respectively from the left and the right ventricles 4b, 4a.

In some examples of the device 1000 and system of the present disclosure, the cardiac assist device 1000 and system 2000 utilize a controller 95 comprising a mechanical pumping mechanism or an oscillator 97, enabling the cardiac loop 60 to move axially and radially between said first position and second position, based on the frequency of the oscillator 97, to mimic the natural contraction of the heart muscles. In some such examples, another contractile force exerted by the cardiac loop 60 or in other words, an additional or a third action carried out by the cardiac loop 60, after or in addition or during the) shortening along both the short axis 14 and long axis 12, is the squeezing action carried out by the cardiac loop 60 against the heart muscle, thereby squeezing the heart muscles thereby mimicking the natural squeezing or torsional or twisting/torquing movement/action (t), as shown in Fig. 1D, by the heart muscle to pump blood to the lungs and the body, respectively. In some such examples, the squeezing action of the heart may be viewed as a torsional or torquing action or movement (t) by the heart muscles, which in some cases may be about or along the longitudinal axis 12 of the heart as shown in Fig. 1D. In some examples of the present disclosure the cardiac loop 60 emulates the natural contraction and/or movement of the heart. In some such examples, the cardiac loop 60 provides a torsional or torquing or twisting movement or action (t) as shown in Fig. 1E, of the cardiac loop 60 that mimics the torsional or torquing action of the heart muscles to pump blood. In some examples of a cardiac assist device 1000 and cardiac assist system 2000 of the present disclosure, a controller 95 comprises a mechanical pumping mechanism or an oscillator 97, enabling the cardiac loop 60 to move substantially torsionally between said first position and second position, based on the frequency of the oscillator 97, in order to mimic the torsional or torquing or twisting/deformation action or movement of the heart, to enable the heart to pump blood. In some such examples, the cardiac loop 60 contracts (axially and radially) and squeezes (torsionally) the heart substantially simultaneously to pump blood to the body and the lungs respectively, from the ventricles 4b, 4a, thereby mimicking the contraction of the heart muscle and the squeezing action of the heart muscle. In some examples of the examples of the present disclosure, the squeezing action may be created through the shortening along both short and long axis 14, 12 as well as by actuation of the cardiac loop 60 by the actuator or oscillator 97 that moves or torques or torsions the cardiac loop during (or in a substantially close time frame to) shortening of the cardiac loop 60 along both short and long axis 14, 12, in a manner that squeezes the heart 2, in order to mimic the natural squeezing motion of the heart 2. In other examples, the squeezing action by the cardiac loop 60 may be created by contracting the cardiac loop 60 both along the short axis 14 and the longitudinal axis 12 of the heart 2.

In accordance with examples of the present disclosure, the cardiac assist device 1000 and/or system 2000 also mimic or simulates the relaxation of the cardiac muscle and the force vector (k) created thereby as the heart 2 relaxes, where the force vector (k) created by the cardiac loop 60 mimics or simulates the vector (k) that created through natural relaxation of the heart 2 or specifically the ventricles 4a, 4b of the heart 2, with both longitudinal/axial force components and radial force components being in the opposite direction to that of force vector (d). The force components thereby, mimicking the relaxed state of the heart during diastole. In one example, Fig, 1F and Fig, 1G, show a location on the heart 2 as shown by a band (B), of a preferred placement of the cardiac loop 60 of a cardiac assist device 1000, in accordance with an example of the present disclosure.

In some examples, one or more sensors 10 are provided on the cardiac loop 60, as shown in Fig. 1C, that measure the ECG signal of the heart. In such examples, the cardiac loop 60 contracts based on feedback from the one or more sensors 10 and enables the contractile rhythm, or in other words the rhythm with which the cardiac loop 60 and as a result the ventricles 4a, 4b are contracting and relaxing, to be synchronized with the existing function and /or electrical rhythm of the heart as measured by the ECG signal. In some examples of the present disclosure the contraction rate and/or rhythm of the contractile force exerted by the cardiac assist device 1000 and/or system 2000 is matched to that of the rhythm of the heart, or specifically the rhythm of ventricular contraction of the heart.

Thus, some examples of the system 2000 of the present disclosure the system 2000 comprises a controller 95 comprising pumping mechanism or actuator or oscillator 97 that for example comprises a motor 99, where the motors 99 allows for contraction and expansion of the cardiac loop 60, that mimics the natural movement of the heart 2, such as the ventricles 4a, 4b, and thus the rhythm of contraction and relaxation of the cardiac loop 60 mimics the systolic rhythm of the heart 2. Thereby the cardiac loop 60, enables the heart muscle to contact upwards from the apex (X) of the heart 2 along the longitudinal axis 12 of the heart 2 or the plane in which the heart lies, as well as radially inwards along the short axis 14 of the heart 2.

Thus, the device and/or systems of the present disclosure mimic the action of the ventricular depolarization that causes the ventricles 4a, 4b to contract. As the ventricles 4a, 4b contract as a result of, the contraction of the cardiac assist device 1000 of the present disclosure, the ventricles 4a, 4b are able to send blood to the body through the aorta and to the lung via the pulmonary artery, with a greater force than can be exerted independently without assistance, by the damaged ventricles of the defective or dysfunctional heart 2. Thus, when the cardiac assist device 1000 is released or relaxes, the atria 3a, 3b fill blood from the lung and the body respectively and contract, thereby sending blood to the ventricles 4a, 4b which then fill with blood.

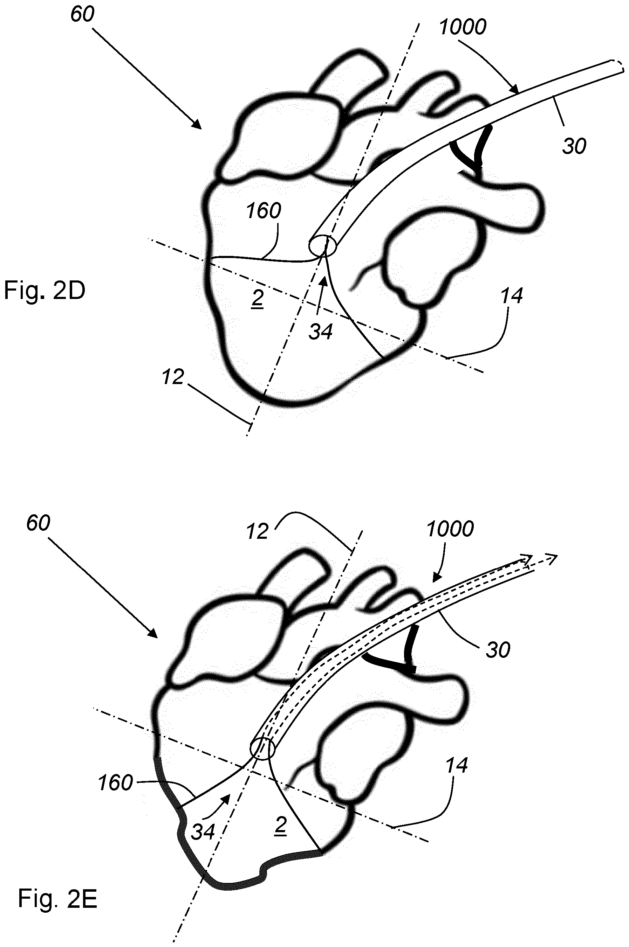

With reference now to Figs. 2A-2E, in some examples, the cardiac assist device 1000 the cardiac loop 60 comprises, a radially contractible and radially expandable cardiac loop 60, as shown in , wherein in said first position 62, the cardiac loop 60 is configured to be in a radially expanded state thereby configured to exert a minimal force on the heart 2, as shown in Fig. 2D, wherein in said second position 64, said cardiac loop 60 is configured to be in a radially contracted state along a short axis 14 of the heart 2, thereby configured to exert a substantial force on the heart 2, to mechanically assist the heart 2 to pump blood, as shown in Fig. 2E.

With reference again to Figs. 2D and 2E, in some such examples of the cardiac assist device 1000, the cardiac loop 60 may also comprise an axially contractible and axially expandable cardiac loop 60, wherein in said first position 62, the cardiac loop 60 is configured to be in an axially expanded state defining a resting state configured to exert a minimal force on the heart 2; and wherein in said second position 64, said cardiac loop 60 is configured to be pulled axially upwards along a longitudinal axis 12 of the heart 2 to move into an axially contracted state, to exert a substantial force on the heart 2 away from the apex (X) of the heart along said longitudinal axis 12, to mechanically assist the heart 2 to pump blood, as shown.

Thus, in some examples of the present disclosure, the cardiac loop 60 may be axially and radially contractible/compressible and expandable. In other examples, the cardiac loop 60 may be axially and/or radially contractible/compressible and expandable. In some such examples of the cardiac assist device 1000, said cardiac loop 60 is radially and axially contractible and expandable, substantially together and/or at substantially at the same time. In some examples of the cardiac assist device 1000, wherein said radially and axially contractible and expandable cardiac loop 60, comprises a line cardiac loop 160 or in other words a line loop or steering line loop 160, formed by said steering line 50. The function of steering line loop 160 or cardiac loop 60 in general is described further herein with respect to Figs. 2A-2E.

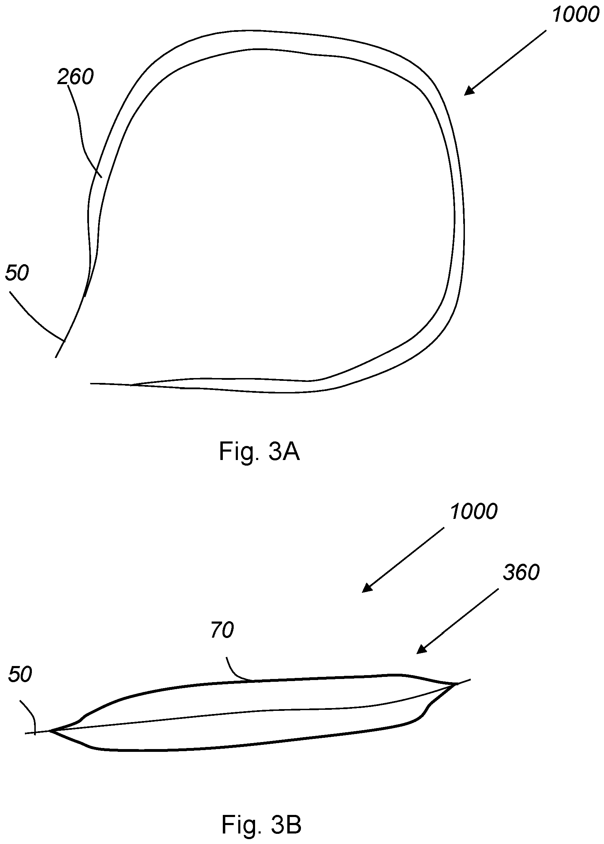

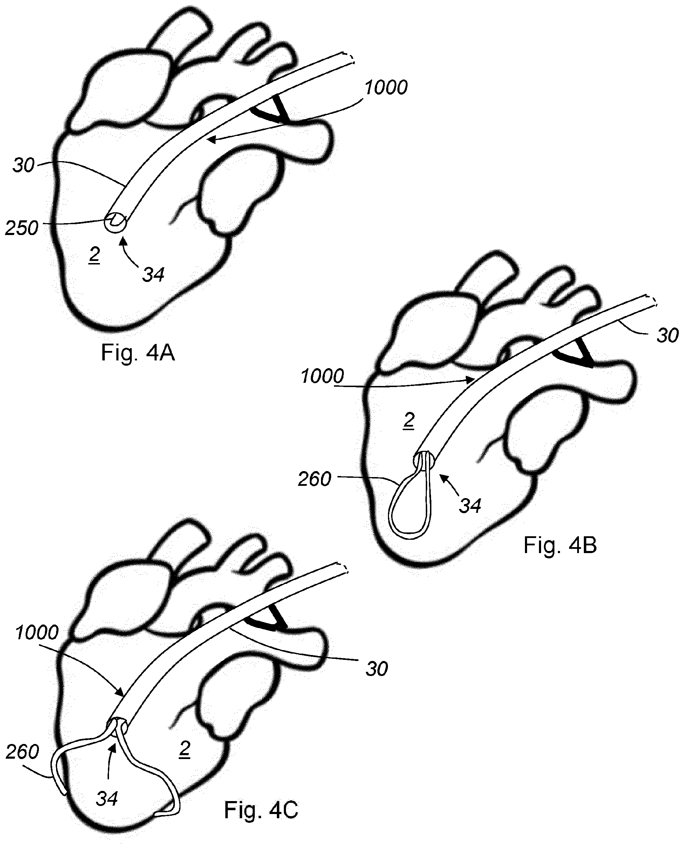

Fig. 3A shows an additional or alternative example of a cardiac assist device 1000 of the present disclosure. The cardiac assist device 1000 comprises a steering line 50, the steering line 50 being hollow, defining a hollow steering line 250. The hollow steering line 250 forms a steering line loop 260. The hollow steering line 250 that has an inflatable part along its length thereof, forming an inflatable cardiac loop 260. The inflatable cardiac loop 260 is deployed such that it straddles the left and right ventricles 4a, 4b of the heart 2. Thus, when the inflatable cardiac loop 260 has been placed around the heart 2, it is inflated such that the diameter of the hollow steering line 250 and hence also the contact surface between the steering line 250 and the heart 2 is increased. Thus, there is a greater contact surface and/or area between the inflatable cardiac loop 260 and the heart 2, will be increased. In some such examples, the hollow steering line 250 may minimize the risk of injury to the heart 2 during use of the device 1000. The function of the inflatable cardiac loop 260 or is described further herein below with respect to Figs. 4A-4E. The inflatable cardiac loop 260 is substantially atraumatic thus preventing and/or minimizing damage to the heart 2 during use.

Thus, in some examples of the cardiac assist device 1000, said radially and axially contractible and radially expandable cardiac loop 60 comprises an inflatable cardiac loop 260 that is configured to engage with and/or coupled to said steering line 50, wherein in said first position the inflatable cardiac loop 260 is configured to have a substantially deflated and expanded profile configured to exert a minimal force on the heart 2 configured to being at rest both axially and radially, and wherein in said second position the inflatable cardiac loop 260 is configured to move axially upwards along the longitudinal axis 12 of the heart and radially inwards along the short axis 14 of the heart to have a substantially inflated and contracted profile configured to exert a substantial force on the heart 2 to mechanically assist the heart 2 in pumping blood.

An additional or alternative example of the cardiac assist device 1000 of the present disclosure is shown in Fig. 3B, which provides the same objective, as described herein above with respect to Fig. 3A. More specifically, the cardiac assist device 1000 provides the advantage of increasing the contact surface between the steering line 50 and the heart 2. In the shown example, a flexible film 70 is attached and rolled onto the steering line 50 along a part of the steering line 50 forming the cardiac loop 60.

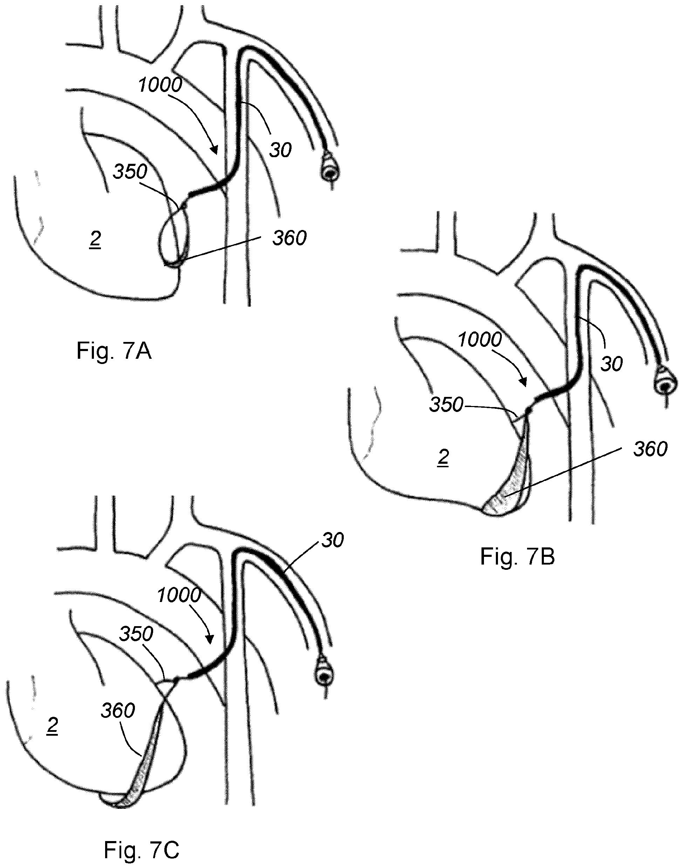

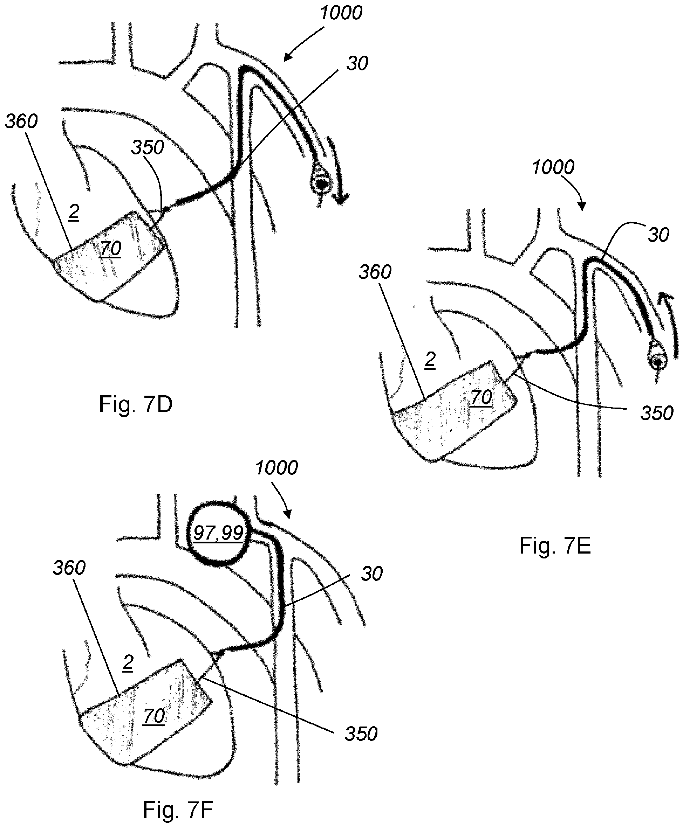

In a further example of the cardiac assist device 1000 of the present disclosure said radially and axially contractible and expandable cardiac loop 60 comprises a flexible cardiac loop or flexible film loop 360, said flexible film loop 360 being formed by a flexible film 70 that is attached to the steering line 50 along a part of the steering line 50, whereby said flexible cardiac loop 360 is configured to be deployed in a substantially flat configuration or state. In some examples, said flexible cardiac loop 360 is radially and axially contractible and expandable in said substantially flat configuration of state. In another example, said flexible cardiac loop 360 is rolled onto the steering line to be attached thereto. The function of the cardiac assist device 1000 comprising the flexible cardiac loop 360 is described further herein below with respect to Figs. 7A-7E. The flexible film loop 360 is substantially atraumatic thus preventing and/or minimizing damage to the heart 2 during use.

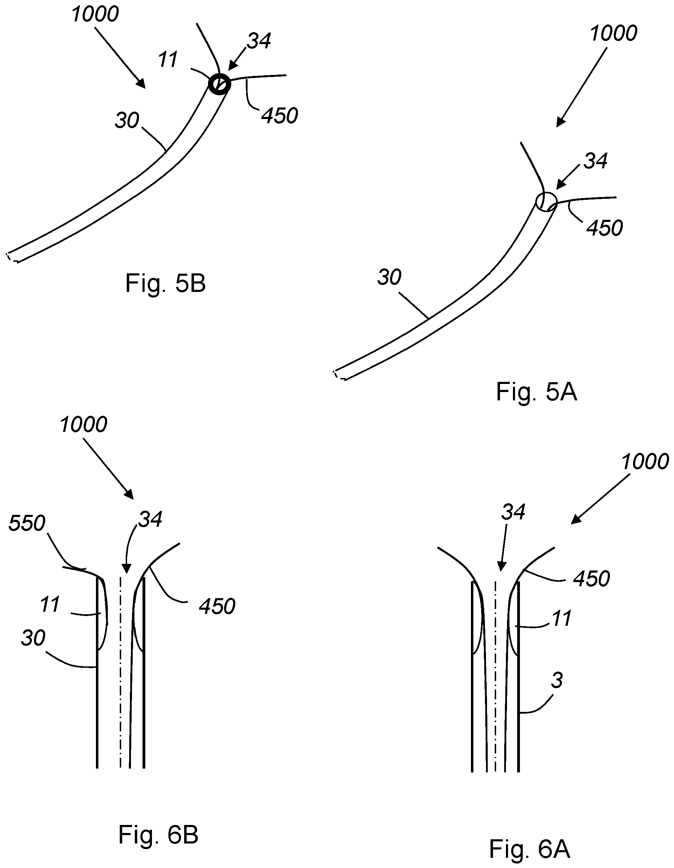

With reference now to Fig. 5A, a cardiac assist device 1000 is shown that does not comprise a reinforcement ring 11. With reference to Fig. 5B, a cardiac assist device 1000 is shown with a reinforcement ring 11. Figs. 6A-6B, show alternate examples of the cardiac assist device 1000 comprising the reinforcement ring 11 that is arranged at the opening 34 of the distal end of the catheter 30, to minimize friction between the catheter 30 and the steering line 50 and/or the cardiac loop 60. Fig. 6A, illustrates an example of the present disclosure wherein one or more ends 450 of the steering line 50 are substantially not attached to the reinforcement ring 11). Fig. 6B, illustrates an example where at least one of the ends 550 of the steering line 50 is attached to the reinforcement ring 11 and/or substantially attached to the reinforcement ring 11.

In some examples of the present disclosure, at least one end of the steering line 50 is arranged through the opening 32 at the proximal end of the catheter 30 (i.e. the proximal opening 32 for controlling the movement of the steering line loop or cardiac loop 60 at the distal end of the catheter 30. In some such examples, two ends of the steering line 50, extend through the catheter 30, from the proximal opening 32. Figure 1C shows one such example where both ends of the steering line 50 are arranged to be controlled at the proximal end of the catheter 30.

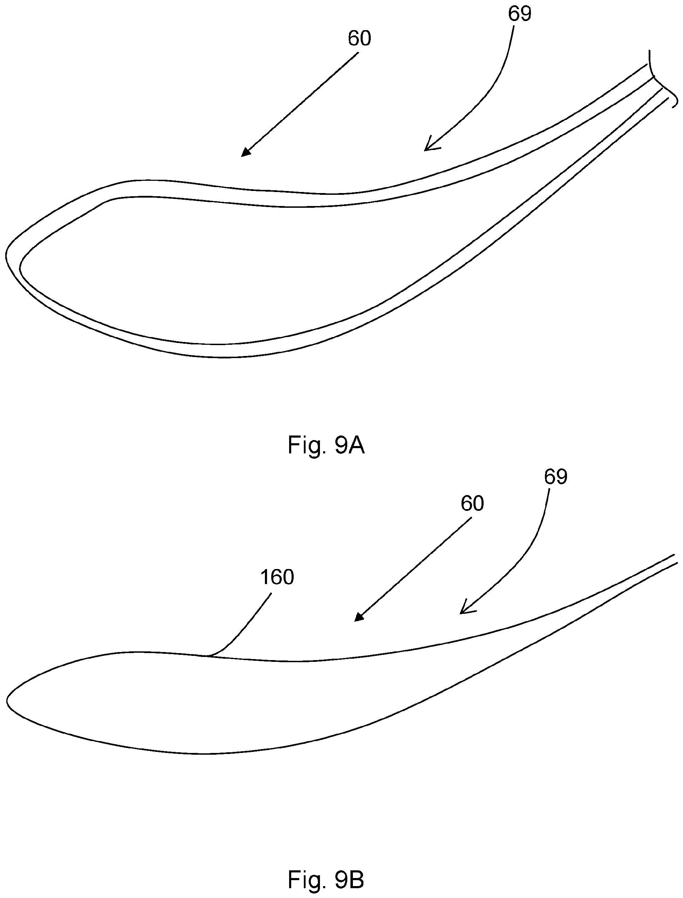

In some examples of the present disclosure, as shown in Fig. 9A, the said cardiac loop 60 of said cardiac assist device 1000 is pre-bent having a substantially boat-shaped configuration 69, to allow said steering line loop to substantially loops around the heart 2 to snare or lasso said heart 2.

With reference to Fig. 9B, said cardiac loop 60 comprises the steering line loop or line loop 160 that comprises nitinol, that is heat-treated to maintain said boat-shaped configuration 69.

### Systems

In some examples of the present disclosure, a cardiac assist system 2000 is disclosed comprising, a cardiac assist device 1000 according to any of the examples described herein above, and a controller 95 for controlling the movement of the cardiac loop 60, said controller 95 enabling axial and radial movement of said cardiac loop 60 between said first position and said second position, to mimic the natural contraction of the heart 2. In some such examples of the cardiac assist system 2000 said controller 95 comprises a mechanical pumping mechanism or an oscillator 97, enabling the cardiac loop 60 to move axially and radially between said first position 62 and said second position 64, based on the frequency of the oscillator 97.

In some such examples of the cardiac assist system 2000, the frequency and/or the amplitude of the mechanical pumping system or oscillator 97 is matched to the natural cardiac rhythm of systole and/or diastole and the contractile force of the heart 2. In some examples of the cardiac assist system 2000, the pumping mechanism or oscillator 97 comprises a motor 99.

In some examples of the cardiac assist system 2000, the cardiac loop 60 comprises one or more sensors 10 for measuring the ECG signal of the heart 2. In one such example, the frequency of the pumping mechanism or oscillator 97 and thus the contraction rate and/or rhythm of the contractile force exerted by the cardiac assist device 1000 on the heart 2 is matched to that of the rhythm of ventricular contraction of the heart 2, based on the ECG signal.

In some examples, the controller 95 and/or the pumping mechanism or the oscillator 97 of the cardiac assist system 2000 is configured to be placed outside the body, (or in other words adapted to be arranged outside of the body). In alternate examples of the cardiac assist system, the controller 95 and/or the pumping mechanism or oscillator 97 is configured to be placed inside the body (or in other words it is adapted to be arranged inside the body).

In additional examples of the cardiac assist device 1000, the controller 97 controls the pumping frequency of the mechanical pumping mechanism 97 or oscillator 97 frequency by controlling a speed of said motor 99. In some example, the controller 97 controls the oscillator 97 amplitude or controls the amplitude of the pumping mechanism 97 through said motor by controlling said motor.

The cardiac assist system of any of the preceding claims as wherein the pumping mechanism and/or the oscillator 97 is attached to the proximal end of the catheter 30 and/or the steering line 50 and arranged such that the steering line is at least pulled in an operating mode by the mechanical pumping mechanism and/or oscillator 97.

In some examples of the cardiac assist system of the present disclosure, wherein at least one end of the steering line 50 is arranged through the opening 32 at the proximal end of the catheter 30 (i.e. the proximal opening 32) to be coupled to said pumping mechanism or oscillator 97, wherein the pumping mechanism or the oscillator 97 controls the movement of the steering line 50 and/or cardiac loop 60 at the distal end of the catheter 30. In some examples, two ends of the steering line 50, extend through the catheter 30, from the proximal opening 32, to be coupled to the pumping mechanism oscillator 97, where the oscillator controls movement of the steering line 50 and/or cardiac loop 60 at the distal end of the catheter 30, by controlling both ends of the steering line 50 at the proximal end of the catheter 30.

In some examples of the cardiac assist system 2000 said motor 99 defined by said pumping mechanism or oscillator 97 is battery powered. In some such examples, the cardiac assist system 2000, further comprising a battery, wherein the battery is adapted to be wirelessly charged.

Thus, with reference to Fig. 1C, a cardiac assist system 2000 of the present disclosure is shown. The cardiac assist system 2000 comprising the catheter device or cardiac assist device 1000, wherein a mechanical pumping mechanism or oscillator 97 is attached to the proximal end of the catheter 30 and/or the steering line 50 and arranged such that the steering line 50 is at least pulled in an operating mode by the mechanical pumping mechanism or mechanical oscillator 97. As noted herein above, according to one example, the pumping mechanism 97 comprises a motor 99.

Thus, Figs. 2A through 2E additionally show a cardiac assist device 1000 that is shown starting with advancing the steering line 50 arranged in a closed loop from the opening 34 at the distal end of the catheter 30. Figs 2B and 2C show further how the steering line loop is arranged around the apex (X) of the heart 2. Fig. 2D shows the cardiac assist device 1000 with the steering line loop 160 around the apex (X) of the heart 2 with the steering line 50 in a relaxed position. In Fig. 2e the steering line 50 is pulled at the proximal end of the catheter 30 such that both the heart is squeezed and the apex (X) is pulled upwards along an axis 12, at substantially the same time.

Further as noted herein above, the motor 99 of the mechanical oscillator or mechanical pumping mechanism 97 could be battery powered and then preferably adapted to be wirelessly charged. In further examples of the system of the present disclosure, a controller or control unit 95, is shown in FIG. 1C in combination with the pumping mechanism or oscillator 97, where the controller or control unit 95 is connected to the pumping mechanism or oscillator 97, in the case of the pumping mechanism or oscillator 97 is a motor 99, for controlling the speed of the motor 99 for controlling the pumping frequency. Furthermore, the controller or control unit 95 is connected to the motor 99 for controlling the amplitude of the pumping.

In some examples, the system shown in FIG. 1C, further shows the steering line loop 160 or cardiac loop 60 comprising one or more sensors 10. The sensors 10 enable measuring of ECG. Thus, in some examples of the present disclosure, this allows for advanced monitoring and adjustment of the contractile rhythm of the cardiac loop 60 or steering line loop 160, wherein the device 1000 and/or the system 2000 provides for automatic adjustment, wherein the device 1000 and/or the system 2000 automatically adjusts to the patient's ECG to ensure optimal support and/or cardiac assistance.

In some examples, the sensors on the cardiac loop 60 can measure temperature, PH, lactate and other parameters as known in the art. In some examples, the cardiac assist system 2000 of the present disclosure, is connected to a server to record patient and motor data, in order to detect any abnormalities, with the consent of the patient. In some examples, the cardiac assist system 2000 may acoustically alarm and call help with GPS information. In some cases, sensors on the loop 60 will automatically trigger an alarm, if contact of the loop 60 with the heart is beginning to diminish.

In accordance with additional or alternative examples of the cardiac assist device 1000 and/or system 2000 of the present disclosure, another example is shown in Figs. 5A and 5B, as also noted herein above, for further minimizing risk during the procedure. More specifically the device and/or system as shown in Figs. 5A and 5B, shows a reinforcement ring 11 that is arranged at the opening 34 of the distal end of the catheter 39, that minimizes the risk of rupture of the distal end of the catheter 30 or the steering line 50 and/or the cardiac loop 60 due to friction between the steering line 50, for example, depending on how long the cardiac assist device 1000 and/or system 2000 is to be used, for example during extended use of the device 1000) and/or system 2000. In some such examples, the steering line 50 may not be coupled to the reinforcement ring 11. Fig. 6A and Fig. 6B, show an additional example where a reinforcement ring 11 is arranged at the opening 34 of the distal end of the catheter 30. Preferably, the reinforcement ring 11 or bushing/sleeve has a rounded inner surface at least at the edge closest to the opening 34 of the distal end of the catheter 30. Preferably, the material used for the reinforcement ring 11 is chosen such that the friction between the reinforcement ring 11 and the steering line 50 is substantially low to minimize the risk of rupture. As can be schematically seen in Figs. 5A, 5B, both ends of the steering line 50 runs through the catheter 30 whereas in Figs. 6A, 6B, one end of the steering line 50 is fixed in the example shown to the reinforcements ring 11. However, one end of the steering line 50 could be fixed to the catheter 30 more or less anywhere.

Thus, with reference to examples of the cardiac assist system 200 as described herein above, further functional and advantages of the system 2000 are described herein below. With reference to Figs. 1A-1C, Figs. 1E-1F, and Figs. 2D and 2E, the cardiac assist system 2000 of the present disclosure is described in further detail herein below. In some examples, the pumping mechanism or oscillator 97 is defined by said motor 99, that enables the steering line 50 and/or the cardiac loop 60 to be moved between the first position 62 and a second position 64, wherein in said first position the cardiac loop 60 such as a steering line loop (160) as disclosed and described herein, substantially straddles the heart 2), substantially without squeezing the heart 2, as shown in Fig. 2D, and wherein in said second position 64 as shown in Fig. 2E, said cardiac loop 60 substantially squeezes the heart axially upwards along a longitudinal axis 12 of the heart and substantially simultaneously squeezes the heart radially inwards along the short axis 14 of the heart 2. In some examples of the cardiac assist system (2000) of the present disclosure, said cardiac loop (60) is configured to move substantially torsionally or in a substantially twisting motion between said first position and second position, thereby squeezing the heart 2, to pump blood to the body and the lung respectfully from the left and right ventricles (4b, 4a).

In some such examples of the system 2000 of the present disclosure, in said second position 64, as shown in Fig. 2E, the pumping mechanism or oscillator 97 as defined by the motor 99, squeezes or compresses the cardiac loop 60 such as the steering line loop 160, by pulling the cardiac loop 60 axially upwards from the apex (X) of the heart 2, along the longitudinal axis 12 of the heart 2 , while simultaneously reducing the diameter of said cardiac loop 60 or steering line loop 160, thereby squeezing or compressing the heart 2, both radially and axially. In some such examples, in said first position 62 as shown in Fig. 2D, the pumping mechanism or oscillator 97as defined by the motor 80, relaxes the cardiac loop 60 or steering line loop 160 by pushing the cardiac loop 60 or steering line loop 160 axially downwards towards the apex (X) of the heart 2 , along the longitudinal axis of the heart 2, while simultaneously increasing the diameter of said cardiac loop 60 or steering line loop 160 so that it substantially matches the outer diameter of the heart 2 in the heart's resting position, thereby relaxing the heart 2 both radially and axially. In examples of the present disclosure the steering line 50 and cardiac loop 60 as wells as the steering line loop 160 are substantially atraumatic to minimize damage to the heart 2 during use.

In some examples, as discussed herein above with reference to Figs. 2A-2E, and with reference to Fig. 1C, the cardiac loop or snare 60 may be a wire in the beginning, or at the start of the procedure, for example forming a steering line loop 160, but is supplanted by a band to facilitate force transmission, for example against the heart. The band may also facilitate in making the steering line loop 160 or cardiac loop 60 more atraumatic during use. In some such examples, the Cardiac assist device (1000) comprises a Bowden cable, defining a catheter or guiding catheter 30 and a steering wire 50, that is slidably received therein, to be advanced there-through during the procedure. After the steering wire 50 and (in some cases the steering wire loop 60 formed therefrom), are transported or advanced through the guiding catheter 30 (preferably of the Bowden cable), a band is formed by inflating a balloon snare with a low pressure, in order to form the loop or cardiac loop 60. Additional or alternatively, the loop or cardiac loop 60 may be formed from nitinol, and is substantially atraumatic. In some such examples, the cardiac loop 60 may comprise nitinol triangles that are atraumatic. In other examples, other materials or other methods may be used to form the loop or cardiac loop 60, that are substantially atraumatic during use.

### Methods

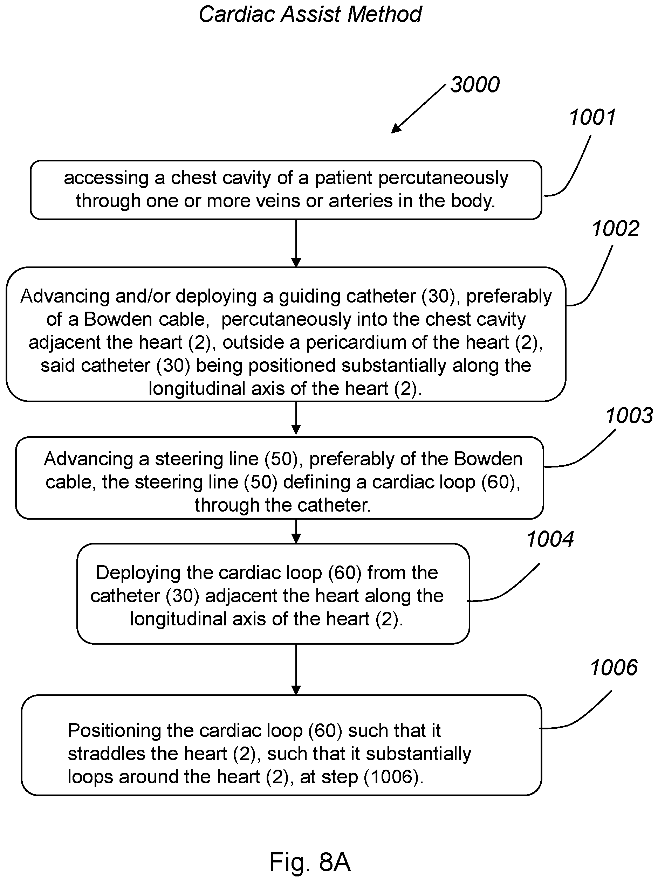

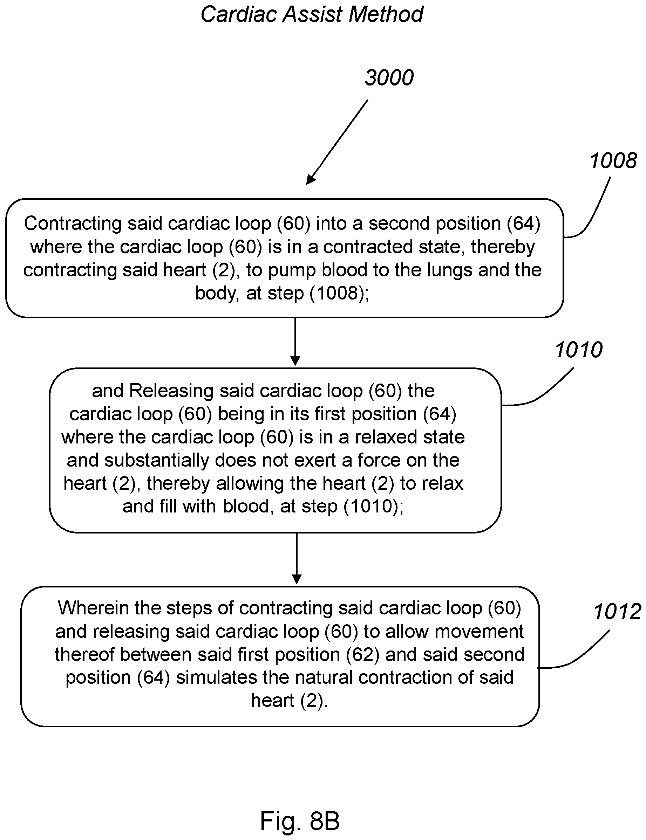

In accordance with an exemplary method 3000 of the present disclosure as shown in Fig. 8A and Fig. 8B, a method is disclosed for assisting the cardiac output of the heart 2 comprises the steps of: accessing a chest cavity of a patient percutaneously through one or more veins or arteries in the body including but not limited to, the radial vein or artery, subclavian vein or artery, jugular vein, carotid artery, femoral and brachial vein or artery, at step 1001; Advancing and/or deploying a catheter 30 (for example preferably a guiding catheter 30 that forms the outer conduit or cable of the Bowden cable) percutaneously into the chest cavity adjacent the heart 2, outside a pericardium of the heart 2, at step 1002, said catheter 30 being positioned substantially along the longitudinal axis of the heart 2; Advancing a steering line 50 defining a cardiac loop 60, through the catheter, at step 1003; Advancing a steering line (50) (for example that preferably forms the inner cable of the Bowden cable and is slidable within the outer cable as defined by the guiding catheter 30 of the present disclosure). In some examples, the steering line (50) defines a cardiac loop (60), that is advanced through the guiding catheter (30), at step (1003). Deploying the cardiac loop 60 from the catheter 30 adjacent the heart along the longitudinal axis of the heart 2, at step 1004; Positioning the cardiac loop 60 such that it straddles the heart 2, such that it substantially loops around the heart 2, at step 1006; Contracting said cardiac loop 60 into a second position 64 where the cardiac loop 60 is in a contracted state, thereby contracting said heart 2, to pump blood to the lungs and the body, at step 1008; and Releasing said cardiac loop 60 the cardiac loop 60 being in its first position 64 where the cardiac loop 60 is in a relaxed state and substantially does not exert a force on the heart 2, thereby allowing the heart 2 to relax and fill with blood, at step 1010; Wherein at step 1012 the steps of contracting said cardiac loop 60 and releasing said cardiac loop 60 to allow movement thereof between said first position 62 and said second position 64 that simulates the natural contraction of said heart 2.

In some such examples, the step of accessing a chest cavity of a patient percutaneously through one or more veins or arteries in the body, at step 1001, further comprises: accessing the chest cavity through one or more of: a radial vein or artery, a cubital vein, a brachial vein or artery, a subclavian vein or artery, a branch of the thoracic vein, and a mammary vein, the jugular vein, the carotid artery, and the femoral vein or artery. The present step is not limited to the above noted veins or arteries.

In some examples, the step of positioning the cardiac loop 60 such that it straddles the heart 2, at step 1006 further comprises: positioning the cardiac loop 60 substantially along the ventricles 4a,4b of the heart 2, such that the cardiac loop 60 snares or lassos the heart 2 along said ventricles 4a, 4b.

In some examples, contracting said cardiac loop 60, at step 1008, further comprises pulling the steering line and/or the cardiac loop 60, thereby shortening the diameter of the cardiac loop 60 to allow ventricles 4a, 4b of the heart 2 to contract substantially radially and axially, thereby simulating the natural contraction of the ventricles 4a, 4b, enabling the ventricles to be placed in a contracted/compresses state, to pump blood to the lungs and the body, the step of releasing said cardiac loop 60, at step 1010, further comprises releasing the steering line and/or the cardiac loop 60 to allow the ventricles 4a, 4b to relax, allowing them to fill with blood.

In some examples the steps of contracting said cardiac loop 60, at step 1008 comprises contracting said cardiac loop 60 radially and/or axially; and the step of releasing said cardiac loop 60, at step 1010comprises expanding said cardiac loop 60 radially and/or axially. More specifically, in such examples, the step of contracting said cardiac loop 60, at step 1008, comprises contracting said cardiac loop 60 both radially inwards along the short axis 14 and axially upwards away from the apex X of the heart 2 along the long axis 12 of the heart 2 to contract said heart.

In further examples, the steps of contracting said cardiac loop 60 radially inwards along the short axis 14, at step 1008, and contracting said cardiac loop 60 axially upwards away from the apex X of the heart 2 along the long axis 12 at step 1008, are performed substantially simultaneously.

In some such examples, of the method of the present disclosure said cardiac loop 60 is pre-bent having a substantially boat-shaped configuration, to allow said cardiac loop 60 to substantially loop around the heart 2 to snare or lasso said heart 2. In one such example said cardiac loop 60 comprises cardiac loop 160 that is heat-treated to maintain said boat-shaped configuration. In some such examples, the cardiac loop 160 comprises nitinol that is heat-treated to maintain said boat-shaped configuration. In some examples of the method of the present disclosure the cardiac loop 60 comprises an inflatable cardiac loop 60 wherein the step of contracting said cardiac loop 60, and releasing said cardiac loop 60, at steps 1008 and1010, further comprise the steps of: inflating said cardiac loop 260 radially at step 1010, thereby squeezing the ventricles along the short axis 12 of the heart 2, along with pulling the cardiac loop 60 axially along the longitudinal axis 14 away from the apex X, at step 2008; and deflating and/or releasing said cardiac loop 260, thereby releasing the squeezing force on the ventricles 4a, 4b along the short axis 12 and the long axis 14 of the heart 2, letting the ventricles 4a,4b return to their resting position at step 2010. In some such examples, said the inflation and deflation of the cardiac loop 260, and thus the rate and/or rhythm of the contractile force exerted by the cardiac loop 260 on the heart 2 is matched to that of the natural ventricular contraction rhythm of the heart 2, for example in systole.

In some examples of the present disclosure, the steps of contracting and releasing said cardiac loop 60 at steps 1008 and 1010, are implemented using a controller 95. As a specific example of this, the steps of contracting and releasing said cardiac loop 60 at steps 1008 and 1010, are performed by a mechanical pumping mechanism or oscillator 97 of the controller 95. In some examples the mechanical pumping mechanism or oscillator 97 defines a motor 99 for carrying out the steps of contracting and releasing said cardiac loop 60.

In some examples of the methods of the present disclosure, the steps of contracting and releasing said cardiac loop 60 at steps 1008 and 1010, are implemented using the motor 99. In some examples of the method of the present disclosure, said motor 99 is implanted internally, within a patient's body. In alternate examples, said motor 99 is implanted externally, outside a patient's body. In some examples of the present method, said steps of contracting said cardiac loop 60 at step 1008, and releasing said cardiac loop 60 at step 1010, are performed over a short-term substantially short period of time, wherein said device 1000 is used to ameliorate or assist in cardiac function on a temporary basis. Alternatively, said steps of contracting said steering line loop 60 at step 1008, and releasing said cardiac loop 60 at step 1010, are performed over a long-term or substantially long period of time, wherein said device 1000 is used on a substantially permanent basis. In some such examples, one or more components of the device 1000 and/or the controller 95 are implanted internally within the body. Additionally or alternatively, in some such examples, one or more components of the device 1000 and/or the controller 95 are positioned external to the body.

In some examples of the method present disclosure, wherein the cardiac loop 60 comprises an elastic cardiac loop 360 in a substantially flat configuration, wherein the method deploying said elastic cardiac loop 360 in said substantially flat configuration, wherein the steps of contracting and releasing said cardiac loop 60 comprises expanding contracting said elastic cardiac loop 360 in said substantially flat configuration.

In some examples of the present disclosure, wherein the method further comprises the steps of: sensing an ECG signal of the heart; and wherein steps of contracting and releasing the cardiac loop 60 and thus the rate and/or rhythm of the contractile force exerted by the cardiac loop 60 is matched to that of the natural contraction rhythm of the heart 2 based on the sensed ECG signal. In some such examples, the method further comprises the steps of: sensing an ECG signal of the heart, and wherein steps of contracting and releasing the cardiac loop 60 and thus the rate and/or rhythm of the contractile force exerted by the cardiac loop 60 is matched to that of the natural systolic ventricular contraction rhythm of the heart 2 based on the sensed ECG signal.

More specifically, with reference to Fig. 1C and Figs. 2A to 2E, the steering line 50 comprises a cardiac loop 60. In some examples of the present disclosure the catheter 30 is deployed longitudinally along the longitudinal axis 12 of the heart, as shown in Fig. 2A. As shown, the steering line 50 is advanced through the catheter 30 and the steering line 50 is then advanced distally, deploying the cardiac loop 60 from the distal opening 34 of the catheter 30, as shown in Fig. 2B. In some examples, thus the cardiac loop 60 is also deployed along the long axis 12 of the heart 2. In some such examples, the cardiac loop 60 of the device 1000, of the present disclosure, is positioned such that it loops around the heart 2 straddling the left and right ventricles 4b, 4a of the heart 2, as shown in Fig. 2C and Fig. 2D.

In alternate examples of a method of the present disclosure, a method of implanting a system for mechanically augmenting a heart 2 is disclosed, the method comprising: entering a catheter 30 from a radial or cubital vessel; advancing the catheter 30 through an opening punctured in a radial access opening or in a cubital vein towards the desired site; advancing a steering line 50 arranged in a closed loop from an opening 34 at the distal end of the catheter 30 and arranging the steering line loop or cardiac loop 60 around the apex X of the heart 2.In some such examples, the method further comprises advancing the catheter 30 through at least a part of a thoracic coronary artery. In some such examples, the method further comprises advancing the catheter 30 through a punctured site in the left thoracic coronary artery or the right thoracic coronary artery.

In some examples of the present disclosure, wherein the steering line 50 is hollow and at least a part of the steering line 50 in the loop formed thereby is elastic, the method further comprising inflating the elastic part of the loop.

In an alternate example of the present disclosure, a method of augmenting a heart 2, is disclosed, the method comprising: arranging a steering line loop 160 around a heart 2; and pulling at least one end of the steering line loop 160 for pumping augmentation of the heart 2 via a catheter 30. In some such examples, the method comprises pushing the at least one end of the steering line loop 160 after each pulling of the end of the at least one end of the steering line loop 160. In some examples, the pulling of the at least one end of the steering line loop 160 is performed manually and externally of the body. In some examples, the pulling of the at least one end of the steering line loop 160 is performed by a motor 99 located inside or outside of the body.

In some examples of the cardiac assist method of the present disclosure, the cardiac assist device 1000 is deployed substantially longitudinally along the longitudinal axis of the heart 2, as shown in Figs. 1A and 1C, such that the cardiac loop 60 straddles the left and right ventricles 4a, 4b of the heart 2. In some examples, the cardiac loop 60 sits around the heart 2 in contact therewith substantially between the atria 3a, 3b and the ventricles 4a, 4b.

Some examples of the present disclosure as outlined herein above, provide a device 1000that comprises a controller 95 for example comprising a mechanical pumping device or oscillator 97, to enable the expansion and contraction of the cardiac loop 60. In some such examples, the oscillator may comprise a motor 99. In some examples the cardiac loop 60 comprises a steering line loop 160 that is that is formed from the steering line 50. The controller 95 and/or the oscillator 97 functions to contract and expands the cardiac loop 60 that is placed around the heart 2, where the movement of the cardiac loop 160 mimics the natural movement of the heart 2, specifically that of the ventricles 4a, 4b and thus, the rhythm of contraction of the cardiac loop 60, mimics the systolic and/or diastolic rhythm of the heart 2.

More specifically, When the cardiac loop 60 is in the expanded state which is the first position 62, the cardiac loop 60 does not substantially apply a force to the heart 2, as shown in Fig. 2D. When the cardiac loop 60 is contracted using the controller 95, the cardiac loop 260 contracts, compressing the heart radially inwards along the short axis 14 of the heart 2, and axially upwards, along the longitudinal axis 12 of the heart 2 away from the apex X of the heart 2, thus mimicking the natural contraction of the ventricles 4a,4b as shown in Fig. 2E.

In some such examples of the method of the present disclosure, the system 2000 of the present disclosure used comprising the mechanical pumping mechanism or oscillator 97comprising a motor 99, that moves the cardiac loop 60 upwards, for example the steering line loop 260. The motor 99 pulls the steerable line loop 260 thereby contracting or compressing it by reducing its diameter radially and by pulling it axially away from the apex of the heart 2 thereby compressing it against the heart 2. As the steering line loop 160 is moved axially upwards along the longitudinal axis 12, it is also radially contracted along the short axis 14. In some examples, the axial movement of the steering line loop 160 and the radial contraction of the steering line loop 160 may occur at substantially the same time, as the steering line loop is shortened and pulled at substantially the same time. This allows the heart muscle to contract upwards along the longitudinal axis of the heart 2 or the plane in which the heart 2 lies away from the apex X of the heart 2, and additionally substantially simultaneously, allows the heart to contract along the short axis 14 of the heart, as shown in Figure 2E. The movement of the cardiac loop 60, or in other words the lasso 60, comprising the steering line loop 160, upwards along the axis 12 of the heart and contraction inwards along the short axis 14 of the heart 2, places a compressive contractile force on the heart 2 that enables simulation of the ventricular action potentials that enable contraction of the ventricles 4a, 4b. As such the cardiac assist method, device and/or systems of the present disclosure, mimics the 3D movement of the heart, or in other words the heart muscle, specifically during ventricular contraction, to pump blood to the lungs for oxygenation and respectively, oxygenated blood to the body.

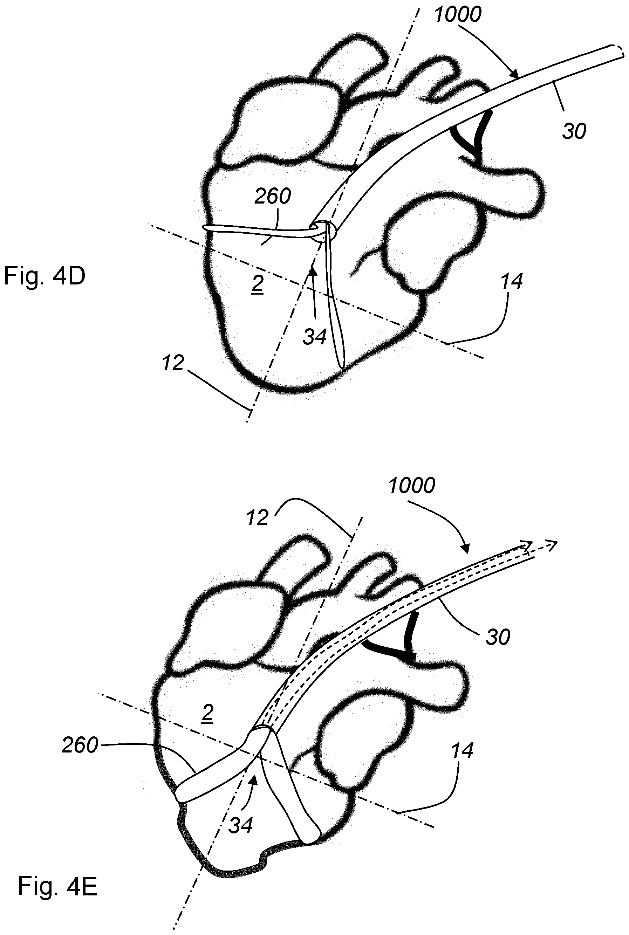

More specifically, with reference to Fig. 3A and Figs. 4A to 4E, the steering line 50 is shown that has an inflatable part. Thus, examples of the present disclosure provide a device 1000 comprising a motor 99 where the expansion and contraction of the cardiac loop 60 comprising an inflatable cardiac loop 260 around the heart 2, mimics the natural movement of the heart 2, specifically that of the ventricles and thus, the rhythm of contraction of the catheter mimics the systolic rhythm of the heart 2. Thus, when the inflatable cardiac loop 260 is inflated, the inflatable cardiac loop 260 expands, compressing the heart radially inwards along the short axis 14 of the heart 2, and axially upwards, along the longitudinal axis 12 of the heart 2, thus mimicking the natural contraction of the ventricles 4a,4b as shown in Fig. 4E. In some examples of the present disclosure the catheter 30 and thus the steering line 50 of the device 1000, is deployed longitudinally along the longitudinal axis 12 of the heart, as shown in Fig. 4A. The steering line 50 is then advanced distally, deploying the inflatable cardiac loop 260 from the distal opening 34 of the catheter 30, as shown in Fig. 4B. In some such examples, the inflatable cardiac loop 260 of the device 1000, of the present disclosure straddles the left ventricle 4b and right ventricle 4a of the heart 2, as shown in Fig. 4C and Fig. 4D.

In accordance with a cardiac assist method of the present disclosure, the system 2000 of the present disclosure used comprising the mechanical pumping mechanism or oscillator 97comprising a motor 99, that moves the inflatable cardiac loop 260 upwards as the inflatable cardiac loop 260 is inflated thereby contracting or compressing it against the heart 2. As the inflatable cardiac loop 260 is moved axially upwards along the longitudinal axis 12, it is also radially contracted along the short axis 14. In some examples, the axial movement of the inflatable cardiac loop 260 and the radial contraction of the inflatable cardiac loop 260 may occur at substantially the same time. This allows the heart muscle to contract upwards along the longitudinal axis of the heart 2 or the plane in which the heart 2 lies away from the apex X of the heart 2, and additionally substantially simultaneously, allows the heart to contract along the short axis 14 of the heart, as shown in Figure 4E. The movement of the cardiac loop 60, or in other words the lasso 60, specifically of the inflatable type comprising the inflatable cardiac loop 260, upwards along the axis 12 of the heart and contraction inwards along the short axis 14 of the heart 2, places a compressive contractile force on the heart 2that enables simulation of the ventricular action potentials that enable contraction of the ventricles 4a, 4b. As such the cardiac assist method, device and/or systems of the present disclosure, mimics the 3D movement of the heart, or in other words the heart muscle, specifically during ventricular contraction, to pump blood to the lungs for oxygenation and respectively, oxygenated blood to the body.

Turning to Figs. 7A through 7F, an example of a cardiac assist method of the present disclosure is shown. The figures illustrate how the cardiac assist system 2000, in accordance with some examples of the present disclosure is deployed in the. In accordance with the method of the present disclosure, a cardiac assist device 1000 comprising the flexible cardiac loop 360 as shown in Fig. 3B, is deployed. The cardiac assist device 1000, specifically a catheter 30 of the cardiac assist device 1000 is advanced through an opening (for example a punctured access point or opening) in a radial vein or in a cubital vein, through the vasculature towards the desired site, adjacent a pericardium of the heart 2, as shown in Fig. 7A. In some examples, the catheter 30 is further advanced through at least a part of a thoracic coronary artery through a punctured site in the left thoracic coronary artery (or the right thoracic coronary artery) to be positioned adjacent the heart 2. Next, a steering line 50 arranged in a closed loop is advanced from an opening 34 at the distal end of the catheter 30, as shown in Fig. 7B. The flexible cardiac loop 360 is then arranged around the apex of the heart 2, as shown in Fig. 7C. In the shown example, with reference to Fig. 3B a flexible film 70 is attached and rolled onto the steering line 50 along a part of the steering line 50 forming the flexible cardiac loop 360 that is deployed in a substantially flat state. Fig. 7D shows the steering line 50 being pulled in order to compress the heart 2. The flexible cardiac loop 360 is pulled axially away from the apex X of the heart thereby compressed axially and is compressed radially inwards, thereby simulating the natural contractile force of the heart 2. In Fig. 7E the steering line 50 is released, and in some examples, may even be pushed in the other direction, i.e., in a direction that is opposite to the contractile force that is exerted by the controller 95. In some such examples, the heart exerts a natural force due to its elasticity to return to its decompressed or relaxed state. In some cases, a battery driven motor 99 together with a controller or control unit 95 is implanted within the patient, so as to perform the pumping action for an extended time of use.

In accordance with any of the examples of the cardiac assist device 1000 of the present disclosure, in one broad aspect, a cardiac assist device 1000 is disclosed, the cardiac assist device comprising: a steering line 50, preferably of a Bowden cable; and a guiding catheter 30, preferably of the Bowden cable, for guiding said steering line 50; said catheter 30 being configured to be advanced percutaneously through vasculature to be positioned along a longitudinal axis of a heart 2 outside a pericardium of said heart 2 inside a patient's body 9000; said steering line being adapted to be received through the catheter and advanced therethrough substantially along said longitudinal axis of said heart 2; said steering line being configured to form a closed loop extending from the distal opening 34 of the catheter 30, thereby forming a cardiac loop 60 for positioning around the heart 2, so that it substantially straddles the heart 2; wherein said cardiac loop 60 is configured to move between a first position and a second position, wherein in said first position the cardiac loop has a substantially enlarged profile, thereby enabling the heart 2 to receive blood and wherein in said second position the cardiac loop 60 has a substantially reduced profile, enabling the heart 2 to contract, thereby assisting the heart 2 to pump blood from the ventricles (4a, 4b), thereby mechanically assisting cardiac function thereby simulating the natural movement of the heart. In accordance with any of the examples of the cardiac assist device 1000 of the present disclosure, the cardiac loop 60 comprises: a radially contractible and radially expandable cardiac loop 60; wherein in said first position cardiac loop 60 is configured to be in a radially expanded state thereby configured to exert a minimal force on the heart 2; and wherein in said second position said cardiac loop 60 is configured to be in a radially contracted state along a short axis 14 of the heart 2, thereby configured to exert a substantial force on the heart 2, to mechanically assist the heart 2 to pump blood.

As an example of this feature, or alternatively as another feature of the broad aspect, the cardiac assist device 1000 is disclosed wherein: said cardiac loop 60 is axially contractible and axially expandable; wherein in said first position cardiac loop 60 is configured to be in an axially expanded state defining a resting state configured to exert a minimal force on the heart 2; and wherein in said second position said cardiac loop 60 is configured to be pulled axially upwards along a longitudinal axis 12 of the heart 2 to move into an axially contracted state, to exert a substantial force on the heart 2 away from the apex X of the heart along said longitudinal axis 12, to mechanically assist the heart 2 to pump blood. In accordance with any of the examples of the cardiac assist device 1000 of the present disclosure, said cardiac loop 60 is radially and axially contractible and expandable, at substantially together and/or at substantially the same time. In accordance with any of the examples of the cardiac assist device 1000 of the present disclosure, said cardiac loop (60) is configured to move substantially torsionally between said first position and second position, wherein said cardiac loop (60) is torsionally contractible and expandable. In accordance with any of the examples of the cardiac assist device 1000 of the present disclosure, the cardiac loop 60 is radially, axially and torsionally, contractible and expandable.

Additionally or alternatively, in some examples, that cardiac loop 60 of the cardiac assist device 1000 has one or more of said features of being radially and/or axially and/or torsionally contractible and expandable. In accordance with any of the examples of the cardiac assist device 1000 of the present disclosure, wherein said cardiac assist device comprises a Bowden cable, wherein said guiding catheter (30) forms and outer cable or conduit of said Bowden cable and said steering line (50) forms an inner cable of said Bowden cable and is slidably receivable within said guiding catheter (30) of said Bowden cable.

In accordance with any of the examples of the cardiac assist device 1000 of the present disclosure, wherein said radially, and/or axially, and/or torsionally contractible and expandable cardiac loop (60), comprises a steering line loop (160), formed by said steering line (50; wherein in said first position steering line loop (160) is configured to be in an axially expanded state defining a resting state configured to exert a minimal force on the heart (2); and wherein in said second position said steering line loop (160) is configured to be pulled axially upwards along a longitudinal axis (12) of the heart (2) to move into an axially contracted state, to exert a substantial force on the heart (2) away from the apex (X) of the heart along said longitudinal axis (12), to mechanically assist the heart (2) to pump blood. In accordance with any of the examples of the cardiac assist device 1000 of the present disclosure, wherein said radially, and/or axially, and/or torsionally contractible and expandable cardiac loop 60 comprises an inflatable cardiac loop 260 that is configured to engage with and/or coupled to said steering line 50; wherein in said first position the inflatable cardiac loop 260 is configured to have a substantially deflated and expanded profile configured to exert a minimal force on the heart 2 configured to being at rest both axially and radially; and wherein in said second position the inflatable cardiac loop 260 is configured to move axially upwards along the longitudinal axis 12 of the heart and radially inwards along the short axis 14 of the heart to have a substantially inflated and contracted profile configured to exert a substantial force on the heart 2 to mechanically assist the heart 2 in pumping blood.

In accordance with any of the examples of the cardiac assist device 1000 of the present disclosure, wherein said radially, and/or axially, and/or torsionally contractable and expandable cardiac loop 60 comprises a flexible film loop 360, said flexible film loop 360 being formed by a flexible film 70 that is attached to the steering line 50 along a part of the steering line 50, whereby said flexible film loop 360 is configured to be deployed in a substantially flat configuration or state; and wherein said flexible film loop 360 is radially and axially contractible and expandable in said substantially flat configuration or state. In accordance with any of the examples of the cardiac assist device 1000 of the present disclosure, at least one end of the steering line 50 is arranged through the opening 32 at the proximal end of the catheter 30 for controlling the movement of the cardiac loop 60 at the distal end of the catheter 30. In accordance with any of the examples of the cardiac assist device 1000 of the present disclosure, both ends of the steering line 50 are arranged to be controlled at the proximal end of the catheter 30, thereby reducing friction between the catheter 30 and the steering line 50 and/or the cardiac loop 60. In accordance with any of the examples of the cardiac assist device 1000 of the present disclosure, wherein said cardiac loop 60 is pre-bent having a substantially boat-shaped configuration, to allow said steering line loop to substantially loops around the heart 2 to snare or lasso said heart 2.

As a feature of this cardiac assist device 1000, said cardiac loop 60 comprises the steering line loop 160 that comprises nitinol that is heat-treated to maintain said boat-shaped configuration. In accordance with any of the examples of the cardiac assist device 1000 of the present disclosure, a reinforcement ring 11 is arranged at the opening 34 of the distal end of the catheter 30, to minimize friction between the catheter 30 and the steering line 50 and/or the cardiac loop 60. As an example of this feature of this cardiac assist device 1000, wherein one or more ends of the steering line 50 are substantially not attached to the reinforcement ring 11. As an example of this feature of this cardiac assist device 1000, wherein at least one of the ends of the steering line 50 is substantially attached to the reinforcement ring 11.

In accordance with any of the examples of the cardiac assist system 2000 of the present disclosure, in one broad aspect, a cardiac assist system 2000 is disclosed comprising: a cardiac assist device 1000 according to any of the examples of the cardiac assist devices 1000 disclosed herein above; and a controller 95 for controlling the movement of the steering line and/or cardiac loop, said controller 95 enabling axial and radial movement of said cardiac loop 60 between said first position and said second position, to mimic the natural contraction of the heart 2. As a feature of this broad aspect, said controller 95 comprises a mechanical pumping mechanism or an oscillator 97, enabling the cardiac loop 60 to move between said first position and second position, based on the frequency of the oscillator 97. As an example of this feature of the cardiac assist system, wherein said controller 95 enables the cardiac loop 60 to move axially and radially between said first position and second position.

As a feature of this cardiac assist system, said controller 95 enables the cardiac loop 60 to move substantially torsionally between said first position and second position. In accordance with any of the examples of the cardiac assist system 2000 of the present disclosure, the frequency and/or the amplitude of the mechanical pumping system or oscillator 97 is matched to the natural cardiac rhythm of systole and/or diastole and the contractile force of the heart 2. In accordance with any of the examples of the cardiac assist system 2000 of the present disclosure, the pumping mechanism or oscillator 97 comprises a motor 99. In accordance with any of the examples of the cardiac assist system 2000 of the present disclosure,, the cardiac loop 60 comprises one or more sensors 10 for measuring the ECG signal of the heart 2 In accordance with any of the examples of the cardiac assist system 2000 of the present disclosure, the frequency of the pumping mechanism or oscillator 97 and thus the contraction rate and/or rhythm of the contractile force exerted by the cardiac assist device 1000 on the heart 2 is matched to that of the rhythm of ventricular contraction of the heart 2, based on the ECG signal. In accordance with any of the examples of the cardiac assist system 2000 of the present disclosure, the controller 95 and/or the pumping mechanism or the oscillator 97 is configured to be placed outside the body.

In accordance with any of the examples of the cardiac assist system 2000 of the present disclosure, the controller 95 and/or the pumping mechanism or oscillator 97 is configured to be placed inside the body. In accordance with any of the examples of the cardiac assist system 2000 of the present disclosure, the controller 97 controls the pumping frequency of the mechanical pumping mechanism 97 or oscillator 97 frequency by controlling a speed of said motor 99. As a feature of any one of the cardiac assist systems 2000 disclosed herein above, wherein the controller 97 controls the oscillator 97 amplitude or controls the amplitude of the pumping mechanism 97 by controlling said motor. In accordance with any of the examples of the cardiac assist system 2000 of the present disclosure, the pumping mechanism and/or the oscillator 97 is attached to the proximal end of the catheter 30 and/or the steering line 50 and arranged such that the steering line is at least pulled in an operating mode by the mechanical pumping mechanism and/or oscillator 97. In accordance with any of the examples of the cardiac assist system 2000 of the present disclosure, wherein at least one end of the steering line 50 is arranged through the opening 32 at the proximal end of the catheter 30 to be coupled to said pumping mechanism or oscillator 97, wherein the pumping mechanism or the oscillator 97 controls the movement of the steering line 50 and/or cardiac loop 60 at the distal end of the catheter 30. In accordance with any of the examples of the cardiac assist system 2000 of the present disclosure, wherein two ends of the steering line 50, extend through the catheter 30, from the proximal opening 32, to be coupled to the pumping mechanism or oscillator 97, wherein the pumping mechanism or oscillator controls movement of the steering line 50 and/or cardiac loop 60 at the distal end of the catheter 30, by controlling both ends of the steering line (50) at the proximal end of the catheter 30. The cardiac assist system in accordance with any of the examples disclosed herein above, wherein said motor 99 defined by said pumping mechanism or oscillator 97 is battery powered. As a feature of this, the cardiac assist system further comprises a battery, wherein the battery is adapted to be wirelessly charged.

In accordance with any of the examples of the cardiac assist method 3000 of the present disclosure, in one broad aspect, a method is disclosed for assisting the cardiac output of the heart 2 comprising the steps of: accessing a chest cavity of a patient percutaneously through one or more veins or arteries in the body, at step 1001, with reference to Fig. 8A; advancing and/or deploying a guiding catheter 30, preferably of a Bowden cable percutaneously into the chest cavity adjacent the heart 2, outside a pericardium of the heart 2, at step 1002, said catheter 30 being positioned substantially along the longitudinal axis of the heart 2; advancing a steering line 50 preferably of the Bowden cable, the steering line 50 defining a cardiac loop 60, through the guiding catheter 30, at step 1003; deploying the cardiac loop 60 from the catheter 30 adjacent the heart along the longitudinal axis of the heart 2, at step 1004; positioning the cardiac loop 60 such that it straddles the heart 2, such that it substantially loops around the heart 2, at step 1006; Contracting said cardiac loop 60 into a second position 64 where the cardiac loop 60 is in a contracted state, thereby contracting said heart 2, to pump blood to the lungs and the body, at step 1008; and releasing said cardiac loop 60, the cardiac loop 60 being in its first position 64, where the cardiac loop 60 is in a relaxed state and substantially does not exert a force on the heart 2, thereby allowing the heart 2 to relax and fill with blood, at step 1010; Wherein the steps of contracting said cardiac loop 60 and releasing said cardiac loop 60 to allow movement thereof between said first position 62 and said second position 64 simulates the natural contraction of said heart 2.

In accordance with any of the examples of the cardiac assist method 3000 of the present disclosure, wherein the step of accessing a chest cavity of a patient percutaneously through one or more veins in the body, at step 1001, further comprises: accessing the chest cavity through one or more of: a radial vein, a cubital vein, a brachial vein or artery, a subclavian vein or artery, a branch of the thoracic vein, and a mammary vein, a jugular vein, a carotid artery, and a femoral vein or artery. As a feature of any of the methods 3000 disclosed herein above, wherein the step of positioning the cardiac loop 60 such that it straddles the heart 2, at step 1006 further comprises: positioning the cardiac loop 60 substantially along the ventricles (4a,4b) of the heart 2, such that the cardiac loop 60 snares or lassos the heart 2 along said ventricles (4a, 4b). As an example of this feature, wherein the method further comprises the steps of: contracting said cardiac loop 60, at step 1008 with reference to Fig. 8B, further comprising the step of pulling the steering line and/or the cardiac loop 60, thereby shortening the diameter of the cardiac loop 60 to allow ventricles (4a, 4b) of the heart 2 to contract substantially radially and axially, thereby simulating the natural contraction of the ventricles (4a, 4b), enabling the ventricles to be placed in a contracted/compresses state, to pump blood to the lungs and the body; and releasing said cardiac loop 60, at step 1010, further comprises releasing the steering line and/or the cardiac loop 60 to allow the ventricles (4a, 4b) to relax, allowing them to fill with blood. In accordance with any of the examples of the cardiac assist method 3000 of the present disclosure, a cardiac assist method 3000 is disclosed, wherein the step of contracting said cardiac loop 60, at step 1008 comprises contracting said cardiac loop 60 radially and/or axially; and wherein the step of releasing said cardiac loop 60, at step 1010 comprises expanding said cardiac loop 60 radially and/or axially. In accordance with any of the examples of the cardiac assist method 3000 of the present disclosure, wherein the step of: contracting said cardiac loop 60, at step 1008, comprises contracting said cardiac loop 60 both radially inwards along the short axis 14 and axially upwards away from the apex X of the heart 2 along the long axis 12 of the heart 2 to contract said heart.

In accordance with any of the examples of the cardiac assist method 3000 of the present disclosure, wherein the steps of contracting said cardiac loop 60 radially inwards along the short axis 14, at step 1008, and contracting said cardiac loop 60 axially upwards away from the apex X of the heart 2 along the long axis 12 at step 1008, are performed substantially simultaneously. In accordance with any of the examples of the cardiac assist method 3000 of the present disclosure, wherein the step of contracting said cardiac loop 60, and releasing said cardiac loop 60, at steps 1008 and 1010, further comprise the steps of: inflating said cardiac loop 260 radially, thereby squeezing the ventricles along the short axis 12 of the heart 2, along with pulling the cardiac loop 60 axially along the longitudinal axis 14 away from the apex X, at step 1008; and deflating and/or releasing said cardiac loop 260, thereby releasing the squeezing force on the ventricles (4a, 4b) along the short axis 12 of the heart 2, letting the ventricles (4a,4b) return to their resting position at step 1010. As a feature of this method, wherein said the inflation and deflation of the cardiac loop 260, and thus the rate and/or rhythm of the contractile force exerted by the cardiac loop 260 on the heart 2 is matched to that of the natural ventricular contraction rhythm of the heart 2, for example in systole. In accordance with any of the examples of the cardiac assist method 3000 of the present disclosure, wherein the method further comprises the steps of: sensing an ECG signal of the heart; and wherein steps of contracting and releasing the cardiac loop 60 and thus the rate and/or rhythm of the contractile force exerted by the cardiac loop 60 is matched to that of the natural contraction rhythm of the heart 2 based on the sensed ECG signal, at step 1012.

A cardiac assist method 3000, in accordance with any of the examples of the method disclosed herein above, wherein the method further comprises the steps of: sensing an ECG signal of the heart; and Wherein steps of contracting and releasing the cardiac loop 60 and thus the rate and/or rhythm of the contractile force exerted by the cardiac loop 60 is matched to that of the natural systolic ventricular contraction rhythm of the heart 2 based on the sensed ECG signal. In accordance with any of the examples of the cardiac assist method 3000 of the present disclosure, wherein steps of contracting and releasing said cardiac loop 60 at steps 1008 and 1010, are implemented using a controller 95. As a feature of this method, wherein the steps of contracting and releasing said cardiac loop 60 at steps 1008 and 1010, are performed by a mechanical pumping mechanism or oscillator 97 of the controller 95. As an example of this method, the steps of contracting and releasing said cardiac loop 60 at steps 1008 and 1010, are performed by a motor 99 defined by said mechanical pumping mechanism or oscillator 97.

As a further example of this method, said motor is implanted internally within a patient's body. As still another example of this method, said motor is positioned externally outside a patient's body. In accordance with any of the examples of the cardiac assist method 3000 of the present disclosure, wherein said steps of: contracting said cardiac loop 60 at step 1008, and releasing said cardiac loop 60 at step 1010, are performed over a short-term or a substantially short period of time, wherein said device 1000 is used to ameliorate or assist in cardiac function on a temporary basis. A cardiac assist method 3000, in accordance with any of the examples of the method disclosed herein above, wherein said steps of contracting said cardiac loop 60 at step 1008, and releasing said cardiac loop 60 at step 1010, are performed over a long-term or substantially longer period of time, wherein said device 1000 is used on a substantially permanent basis. In accordance with any of the examples of the cardiac assist method 3000 of the present disclosure, wherein said steps of wherein said cardiac loop 60 is pre-bent having a substantially boat-shaped configuration, to allow said cardiac loop 60 to substantially loop around the heart 2 to snare or lasso said heart 2. As a feature of this method, wherein said cardiac loop 60 comprises cardiac loop 160 comprises nitinol that is heat-treated to maintain said boat-shaped configuration. In accordance with any of the examples of the cardiac assist method 3000 of the present disclosure, wherein the cardiac loop 60 comprises an elastic cardiac loop 360 in a substantially flat configuration, wherein the method deploying said elastic cardiac loop 360 in said substantially flat configuration, wherein the steps of contracting and releasing said cardiac loop (60) comprises expanding contracting said elastic cardiac loop 360 in said substantially flat configuration. In accordance with any of the examples of the cardiac assist method 3000 of the present disclosure, a method is disclosed, of implanting a system for mechanically augmenting a heart 2, comprising: entering a catheter 30 from a radial or cubital vessel; advancing the catheter 30 through an opening punctured in a radial access opening or in a cubital vein towards the desired site; advancing a steering line 50 arranged in a closed loop from an opening 34 at the distal end of the catheter 30; arranging the steering line loop or cardiac loop 60 around the apex X of the heart 2. In accordance with any of the examples of the methods of the present disclosure, the method further comprises advancing the catheter (30) through at least a part of a thoracic artery.

As an example of this method, further comprising advancing the catheter 30 through a punctured site in the left thoracic coronary artery or the right thoracic artery. In accordance with any of the examples of the methods 3000 of the present disclosure, wherein the steering line 50 is hollow and at least a part of the steering line 50 in the loop formed thereby is elastic, the method further comprising inflating the elastic part of the loop. In accordance with any of the examples of the cardiac assist method 3000 of the present disclosure, a method of augmenting a heart 2 is disclosed, comprising: (i) arranging a steering line loop 160 around a heart 2; and (ii) pulling at least one end of the steering line loop 160 for pumping augmentation of the heart 2 via a catheter 30. In accordance with any of the examples of the cardiac assist method 3000 of the present disclosure, the method further comprising pushing the at least one end of the steering line loop 160 after each pulling of the end of the at least one end of the steering line loop 160. In accordance with any of the examples of the cardiac assist method 3000 of the present disclosure, wherein the pulling of the at least one end of the steering line loop 160 is performed manually and externally of the body. In accordance with any of the methods discussed herein above, wherein the pulling of the at least one end of the steering line loop 160 is performed by a motor 99 located inside or outside of the body.

In one broad aspect, a cardiac assist device (1000) is disclosed, the cardiac assist device comprising: a steering line (50); and a guiding catheter (30), preferably forming an outer cable or conduit of said Bowden cable for guiding said steering line (50); and said steering line (50), preferably forming an inner cable of said Bowden cable and is slidably receivable within said guiding catheter (30) of said Bowden cable; said catheter (30) being configured to be advanced percutaneously through vasculature including one or more veins and/or arteries to be positioned along a longitudinal axis of a heart (2) outside a pericardium of said heart (2); said steering line being adapted to be received through the catheter (30) and advanced therethrough substantially along said longitudinal axis of said heart (2); said steering line being configured to form a closed loop extending from the distal opening (34) of the catheter (30), thereby forming a cardiac loop (60) for positioning around the heart (2), so that it substantially straddles the heart (2); wherein said cardiac loop (60) is configured to contract, thereby contracting the heart (2) assisting the heart (2) to pump blood, thereby mechanically assisting cardiac function.

As a feature of this broad aspect, the cardiac assist device (1000) of claim 1, wherein the cardiac loop (60) comprises: a radially contractible cardiac loop (60); the radially contractible cardiac loop (60) being configured to contract radially along said short axis (14) of the heart (2) to exert a substantial radially directed force on the heart (2), to mechanically assist the heart (2) to pump blood. The cardiac assist device (1000) of any of the previous examples, wherein: said cardiac loop (60) comprises an axially contractible cardiac loop (60); wherein said axially contractible cardiac loop (60) being configured to contract axially to exert a substantial force on the heart (2) away from the apex (X) of the heart along said longitudinal axis (12) of the heart (2), to mechanically assist the heart (2) to pump blood.

The cardiac assist device (1000) of any of the previous examples, wherein said cardiac loop (60) is a torsionally contractible cardiac loop (60); said cardiac loop (60) being configured to contract torsionally along the longitudinal axis (12) of the heart (2) to exert a torsional force against said heart (2) to assist the, to mechanically assist the heart (2) to pump blood. The cardiac assist device (1000) of any of the previous examples, wherein said cardiac loop (60) comprises a steering line loop (160) formed by said steering line (50) wherein said steering line loop (160) is configured to contract through action of the Bowden cable. The cardiac assist device (1000) of any of the previous examples, wherein said cardiac loop (60) comprises an inflatable cardiac loop (260) that is configured to engage with and/or coupled to said steering line (50); said inflatable cardiac loop (260) being configured to be inflated to contract axially, radially or torsionally to contract to the heart (to mechanically assist the heart (2) in pumping blood.

The cardiac assist (1000) of any of the previous examples, wherein said cardiac loop (60) comprises: a flexible film loop (360), said flexible film loop (360) being formed by a flexible film (70) that is coupled to the steering line (50) along a part of the steering line (50), said flexible film loop (306) having a substantially flat configuration or state; and said flexible film loop (360) being configured to contract radially, axially or torsionally to contract the heart to mechanically assist the heart (2) in pumping blood. The cardiac assist device of any of the previous examples wherein at least one end of the steering line (50) is arranged through an opening 32 at the proximal end of the catheter (30) for controlling the movement of the cardiac loop (60) at the distal end of the catheter (30). The cardiac assist device (1000) of any of the previous examples, wherein said cardiac loop (60) is pre-bent having a substantially boat-shaped configuration, to allow said cardiac loop (60) to substantially loop around the heart (2) to snare or lasso said heart (2); or wherein said cardiac loop (60) comprises the steering line loop (160) that comprises nitinol that is heat-treated to maintain said boat-shaped configuration.

In one broad aspect, a cardiac assist system (2000) is disclosed comprising: a cardiac assist device (1000) according to any of the previous examples; and a controller (95) for controlling the movement of the steering line and/or cardiac loop, said controller (95) enabling axial and radial movement of said cardiac loop (60) between said first position and said second position, to mimic the natural contraction of the heart (2). As a feature of this broad aspect, said controller (95) comprises: an oscillator (97);wherein at least one end of the steering line (50) is arranged through the opening 32 at the proximal end of the catheter (30) to be coupled to the oscillator for controlling the movement of the cardiac loop (60) at the distal end of the catheter (30);said oscillator being configured to move and/or contract said cardiac loop (60) axially and radially or torsionally to contract said heart (2) based on the frequency of the oscillator (97). The cardiac assist system (2000) of any of the previous examples of the system, wherein the frequency and/or the amplitude of the oscillator (97) is matched to the natural cardiac rhythm of systole and/or diastole and the contractile force of the heart (2) or wherein the cardiac loop (60) comprises one or more sensors (10) for measuring the ECG signal of the heart (2), wherein the frequency of the pumping mechanism or oscillator (97) and thus the contraction rate and/or rhythm of the contractile force exerted by the cardiac assist device (1000) on the heart (2) is matched to that of the rhythm of ventricular contraction of the heart (2), based on the ECG signal.

In one broad aspect, a method is disclosed for assisting the cardiac output of the heart (2) comprising the steps of: accessing a chest cavity of a patient percutaneously through one or more veins or arteries in the body; advancing and/or deploying a guiding catheter (30), of a Bowden cable percutaneously into the chest cavity adjacent the heart (2), outside a pericardium of the heart (2), said catheter (30) being positioned substantially along the longitudinal axis of the heart (2); advancing a steering line (50) of the Bowden cable, the steering line (50) defining a cardiac loop (60), through the guiding catheter (30); deploying the cardiac loop (60) from the catheter (30) adjacent the heart along the longitudinal axis of the heart (2); positioning the cardiac loop (60) such that it straddles the heart (2), such that it substantially loops around the heart (2); contracting said cardiac loop (60) into a second position (64) where the cardiac loop (60) is in a contracted state, thereby contracting said heart (2), to pump blood to the lungs and the body; and releasing said cardiac loop (60) the cardiac loop (60) being in its first position (64) where the cardiac loop (60) is in a relaxed state and substantially does not exert a force on the heart (2), thereby allowing the heart (2) to relax and fill with blood; thereby simulating the natural contraction and relaxation of said heart (2).As a feature of this broad aspect, wherein the step of accessing a chest cavity of a patient percutaneously through one or more veins or arteries in the body further comprises: accessing the chest cavity through one or more of: a radial vein, a cubital vein, a brachial vein or artery, a subclavian vein or artery, a jugular vein, a carotid artery, a femoral vein or artery, a branch of the thoracic vein, and a mammary vein.

In one broad aspect, a software is disclosed for assisting cardiac function, that includes code segments for performing any of the methods as described herein above, preferably stored on a computer readable medium, preferably for processing by the controller of the system of any of the examples disclosed herein above.

In one broad aspect, a cardiac assist device is disclosed, the cardiac assist device comprising: a steering line; and a guiding catheter for guiding said steering line; said catheter being configured to be advanced percutaneously through vasculature comprising one or more veins and/or arteries, to be positioned along a longitudinal axis of a heart outside a pericardium of said heart inside a patient's body; said steering line being adapted to be received through the catheter and advanced therethrough substantially along said longitudinal axis of said heart; said steering line being configured to form a closed loop extending from the distal opening of the catheter, thereby forming a cardiac loop for positioning around the heart, so that it substantially straddles the heart; wherein said cardiac loop is configured to move between a first position and a second position, wherein in said first position the cardiac loop has a substantially enlarged profile, thereby enabling the heart to receive blood and wherein in said second position the cardiac loop has a substantially reduced profile, enabling the heart to contract, thereby assisting the heart to pump blood, thereby mechanically assisting cardiac function thereby simulating the natural movement or contractile rhythm of the heart.

As a feature of this broad aspect of the cardiac assist device, the cardiac loop comprises: a radially contractible and radially expandable cardiac loop; wherein in said first position cardiac loop is configured to be in a radially expanded state thereby configured to exert a minimal force on the heart; and wherein in said second position said cardiac loop is configured to be in a radially contracted state along a short axis of the heart, thereby configured to exert a substantial force on the heart, to mechanically assist the heart to pump blood. As an example of this feature of the cardiac assist device, said cardiac loop is axially contractible and axially expandable; wherein in said first position cardiac loop is configured to be in an axially expanded state defining a resting state configured to exert a minimal force on the heart; and wherein in said second position said cardiac loop is configured to be pulled axially upwards along a longitudinal axis of the heart to move into an axially contracted state, to exert a substantial force on the heart away from the apex of the heart along said longitudinal axis, to mechanically assist the heart to pump blood. As a further example of above features of the cardiac assist device, said cardiac loop is torsionally contractible and expandable, wherein said cardiac loop is configured to move substantially torsionally between said first position and second position. As a further example of above features of the cardiac assist device, said cardiac assist device comprises a Bowden cable, wherein: said guiding catheter forms an outer cable or conduit of said Bowden cable; and said steering line forms an inner cable of said Bowden cable and is slidably receivable within said guiding catheter of said Bowden cable. As a further example of above features of the cardiac assist device, said cardiac loop comprises a steering line loop formed by said steering line wherein said steering line loop is moveable between said first position and said second position through action of the Bowden cable.

As a further example of above features of the cardiac assist device, said cardiac loop comprises an inflatable cardiac loop that is configured to engage with and/or coupled to said steering line; wherein in said first position the inflatable cardiac loop is configured to have a substantially deflated and expanded profile configured to exert a minimal force on the heart configured to being at rest both axially and radially and/or torsionally; and wherein in said second position the inflatable cardiac loop is configured to move axially upwards along the longitudinal axis of the heart and radially inwards along the short axis of the heart and/or contracting torsionally to have a substantially inflated and contracted profile configured to exert a substantial force on the heart to mechanically assist the heart in pumping blood. As a further example of above features of the cardiac assist device, said cardiac loop comprises a flexible film loop, said flexible film loop being formed by a flexible film that is attached to the steering line along a part of the steering line, whereby said flexible film loop is configured to be deployed in a substantially flat configuration or state; and wherein said flexible film loop is radially and axially and/or torsionally contractible and expandable in said substantially flat configuration or state. As a further example of above features of the cardiac assist device, wherein said cardiac loop is pre-bent having a substantially boat-shaped configuration, to allow said cardiac loop to substantially loop around the heart to snare or lasso said heart. As an example of this feature of the cardiac assist device, wherein said cardiac loop comprises the steering line loop that comprises nitinol that is heat-treated to maintain said boat-shaped configuration.

In one broad aspect, a cardiac assist system is disclosed comprising: a cardiac assist device according to any of the preceding claims; and a controller for controlling the movement of the steering line and/or cardiac loop, said controller enabling axial and radial movement of said cardiac loop between said first position and said second position, to mimic the natural contraction of the heart. As a feature of this broad aspect of the cardiac assist system, wherein said controller comprises: a mechanical pumping mechanism or an oscillator; wherein at least one end of the steering line is arranged through the opening at the proximal end of the catheter to be coupled to a controller for controlling the movement of the cardiac loop at the distal end of the catheter, enabling the controller to move cardiac loop axially and radially and/or torsionally between said first position and second position, based on the frequency of the oscillator. As an example of this feature of the cardiac assist system, the frequency and/or the amplitude of the mechanical pumping system or oscillator is matched to the natural cardiac rhythm of systole and/or diastole and the contractile force of the heart.

As a further example of above features of the cardiac assist system, the cardiac loop comprises one or more sensors for measuring the ECG signal of the heart; wherein the frequency of the pumping mechanism or oscillator and thus the contraction rate and/or rhythm of the contractile force exerted by the cardiac assist device on the heart is matched to that of the rhythm of ventricular contraction of the heart, based on the ECG signal.

In one broad aspect, a method is disclosed for assisting the cardiac output of the heart comprising the steps of: accessing a chest cavity of a patient percutaneously through one or more veins or arteries in the body, at step (1001); advancing and/or deploying a guiding catheter, of a Bowden cable percutaneously into the chest cavity adjacent the heart, outside a pericardium of the heart, at step (1002), said catheter being positioned substantially along the longitudinal axis of the heart; advancing a steering line of the Bowden cable, the steering line defining a cardiac loop, through the guiding catheter, at step (1003); deploying the cardiac loop from the catheter adjacent the heart along the longitudinal axis of the heart, at step (1004); positioning the cardiac loop such that it straddles the heart, such that it substantially loops around the heart, at step (1006); Contracting said cardiac loop into a second position where the cardiac loop is in a contracted state, thereby contracting said heart, to pump blood to the lungs and the body, at step (1008); and Releasing said cardiac loop the cardiac loop being in its first position where the cardiac loop is in a relaxed state and substantially does not exert a force on the heart, thereby allowing the heart to relax and fill with blood, at step (1010); wherein the steps of contracting said cardiac loop and releasing said cardiac loop to allow movement thereof between said first position and said second position simulates the natural contraction of said heart (2).

As a feature of this broad aspect, the step of accessing a chest cavity of a patient percutaneously through one or more veins or arteries in the body, at step (1001), further comprises: accessing the chest cavity through one or more of: a radial vein, a cubital vein, a brachial vein or artery, a subclavian vein or artery, a jugular vein, a carotid artery, a femoral vein or artery, a branch of the thoracic vein, and a mammary vein. As an example of this feature, the step of positioning the cardiac loop such that it straddles the heart, at step (1006) further comprises: positioning the cardiac loop substantially between the atria and the ventricles substantially along the ventricles of the heart, such that the cardiac loop snares or lassos the heart along said ventricles. As a further example of above features of the cardiac assist method, the method further comprises the steps of: contracting said cardiac loop, at step (1008), further comprises pulling the steering line and/or the cardiac loop, thereby shortening the cardiac loop radially inwards along the short axis and axially upwards away from the apex of the heart along the long axis of the heart and/or torsionally along the long axis, thereby contracting the cardiac loop to allow ventricles of the heart to contract substantially radially and axially and/or torsionally; thereby simulating the natural contraction of the ventricles, enabling the ventricles to be placed in a contracted or compresses state, to pump blood to the lungs and the body, thereby mimicking systolic rhythm of the heart; and releasing said cardiac loop, at step (1010), further comprises releasing the steering line and/or the cardiac loop to allow the ventricles to relax, thereby mimicking diastolic rhythm of the heart, allowing them to fill with blood from the atria.

As a further example of above features of the cardiac assist method, the contraction and relaxation of the cardiac loop, and thus the rate and/or rhythm of the contractile force exerted by the cardiac loop on the heart is matched to that of the natural ventricular contraction rhythm of the heart, for example in systole. As a further example of above features of the cardiac assist method, the method further comprises the steps of sensing an ECG signal of the heart; and wherein steps of contracting and releasing the cardiac loop and thus the rate and/or rhythm of the contractile force exerted by the cardiac loop is matched to that of the natural systolic ventricular contraction rhythm of the heart based on the sensed ECG signal.

As used wherein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present. Furthermore, "connected" or "coupled" as used herein may include wirelessly connected or coupled. As used herein, the term "and/or" includes any and all combinations of one or more of the associated listed items.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein. Examples of the present disclosure are described herein with reference to flowchart and/or block diagrams. It will be understood that some or all of the illustrated blocks may be implemented by computer program instructions. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

While several examples of the present disclosure have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present disclosure. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used.

### REFERENCE NUMBERS

(2) heart
apex (X) of the heart (2)
longitudinal axis (12)
short axis (24)
(4a) right ventricle
(4b) left ventricle
(2000) cardiac assist system
   (1000) cardiac assist device
      (50) steering line
      (450) ends of the steering line
      (11) reinforcement ring
      (550) steering line attached to ring (11)
         (60) cardiac loop
         (160) steering line loop or line cardiac loop
   (30) catheter
      (32) proximal opening
      (34) distal opening

      (160) steering line loop or line cardiac loop
      (260) inflatable cardiac loop
      (360) flexible cardiac loop
(95) Controller
   (97) mechanical pumping means or oscillator
   (99) motor
(69) boat-shaped configuration
(1001), (1002), (1003), (1004), (1006), (1008) method steps

## Claims

1. A cardiac assist device (1000), the cardiac assist device comprising:
a steering line (50); and
a guiding catheter (30), preferably forming an outer cable or conduit of said Bowden cable for guiding said steering line (50);
and said steering line (50), preferably forming an inner cable of said Bowden cable and is slidably receivable within said guiding catheter (30) of said Bowden cable;
said catheter (30) being configured to be advanced percutaneously through vasculature including one or more veins and/or arteries to be positioned along a longitudinal axis of a heart (2) outside a pericardium of said heart (2);
said steering line being adapted to be received through the catheter (30) and advanced therethrough substantially along said longitudinal axis of said heart (2);
said steering line being configured to form a closed loop extending from the distal opening (34) of the catheter (30), thereby forming a cardiac loop (60) for positioning around the heart (2), so that it substantially straddles the heart (2);
wherein said cardiac loop (60) is configured to contract, thereby contracting the heart (2) assisting the heart (2) to pump blood, thereby mechanically assisting cardiac function.

2. The cardiac assist device (1000) of claim 1, wherein the cardiac loop (60) comprises:
a radially contractible cardiac loop (60);
the radially contractible cardiac loop (60) being configured to contract radially along said short axis (14) of the heart (2) to exert a substantial radially directed force on the heart (2), to mechanically assist the heart (2) to pump blood.

3. The cardiac assist device (1000) of any of the preceding claims, wherein:
said cardiac loop (60) comprises an axially contractible cardiac loop (60);
wherein said axially contractible cardiac loop (60) being configured to contract axially to exert a substantial force on the heart (2) away from the apex (X) of the heart along said longitudinal axis (12) of the heart (2), to mechanically assist the heart (2) to pump blood.

4. The cardiac assist device (1000) of any of the preceding claims, wherein said cardiac loop (60) is a torsionally contractible cardiac loop (60); said cardiac loop (60) being configured to contract torsionally along the longitudinal axis (12) of the heart (2) to exert a torsional force against said heart (2) to assist the, to mechanically assist the heart (2) to pump blood.

5. The cardiac assist device (1000) of any of the preceding claims, wherein said cardiac loop (60) comprises a steering line loop (160) formed by said steering line (50) wherein said steering line loop (160) is configured to contract through action of the Bowden cable.

6. The cardiac assist device (1000) of any of the preceding claims, wherein said cardiac loop (60) comprises an inflatable cardiac loop (260) that is configured to engage with and/or coupled to said steering line (50);
said inflatable cardiac loop (260) being configured to be inflated to contract axially, radially or torsionally to contract to the heart (to mechanically assist the heart (2) in pumping blood.

7. The cardiac assist (1000) of any of the preceding claims, wherein said cardiac loop (60) comprises:
a flexible film loop (360), said flexible film loop (360) being formed by a flexible film (70) that is coupled to the steering line (50) along a part of the steering line (50), said flexible film loop (306) having a substantially flat configuration or state; and
said flexible film loop (360) being configured to contract radially, axially or torsionally to contract the heart to mechanically assist the heart (2) in pumping blood.

8. The cardiac assist device of any of the preceding claims wherein at least one end of the steering line (50) is arranged through an opening 32 at the proximal end of the catheter (30) for controlling the movement of the cardiac loop (60) at the distal end of the catheter (30).

9. The cardiac assist device (1000) of any of the preceding claims, wherein said cardiac loop (60) is pre-bent having a substantially boat-shaped configuration, to allow said cardiac loop (60) to substantially loop around the heart (2) to snare or lasso said heart (2); or wherein said cardiac loop (60) comprises the steering line loop (160) that comprises nitinol that is heat-treated to maintain said boat-shaped configuration.

10. A cardiac assist system (2000) comprising:
a cardiac assist device (1000) according to any of the preceding claims; and
a controller (95) for controlling the movement of the steering line and/or cardiac loop, said controller (95) enabling axial and radial movement of said cardiac loop (60) between said first position and said second position, to mimic the natural contraction of the heart (2).

11. The cardiac assist system (2000) of claim 10, wherein said controller (95) comprises: an oscillator (97);
wherein at least one end of the steering line (50) is arranged through the opening 32 at the proximal end of the catheter (30) to be coupled to the oscillator for controlling the movement of the cardiac loop (60) at the distal end of the catheter (30);said oscillator being configured to move and/or contract said cardiac loop (60) axially and radially or torsionally to contract said heart (2) based on the frequency of the oscillator (97).

12. The cardiac assist system (2000) of any one of claims 11 to 12, wherein the frequency and/or the amplitude of the oscillator (97) is matched to the natural cardiac rhythm of systole and/or diastole and the contractile force of the heart (2) or
wherein the cardiac loop (60) comprises one or more sensors (10) for measuring the ECG signal of the heart (2), wherein the frequency of the pumping mechanism or oscillator (97) and thus the contraction rate and/or rhythm of the contractile force exerted by the cardiac assist device (1000) on the heart (2) is matched to that of the rhythm of ventricular contraction of the heart (2), based on the ECG signal.

13. A software for assisting cardiac function, in particular for assisting the cardiac output of the heart (2) comprising code segments for performing a method including processing by the controller of the system of any one of claims 10 to 12, for
- contracting cardiac loop (60) into a contracted position (64) where the cardiac loop (60) is in a contracted state, thereby contracting said heart (2), to pump blood to the lungs and the body; and
- releasing said cardiac loop (60) the cardiac loop (60) being in its first position (64) where the cardiac loop (60) is in a relaxed state and substantially does not exert a force on the heart (2), thereby allowing the heart (2) to relax and fill with blood;
thereby simulating the natural contraction and relaxation of said heart (2), wherein
- a steering line (50) of a Bowden cable defining said cardiac loop (60), and said cardiac loop (60) delivered in a chest cavity of a patient from a catheter (30) adjacent the heart along a longitudinal axis of the heart (2), and said cardiac loop (60) being positioned such that it straddles the heart (2), such that it substantially loops around the heart (2).

14. The software of claim 13, wherein said chest cavity of a patient is percutaneously accessed through one or more veins or arteries in the body, including through one or more of: a radial vein, a cubital vein, a brachial vein or artery, a subclavian vein or artery, a jugular vein, a carotid artery, a femoral vein or artery, a branch of the thoracic vein, and a mammary vein.

15. The software for assisting cardiac function stored on a computer readable medium.
